# EUROPEAN PATENT APPLICATION

(11) **EP 4 711 422 A1**
(43) Date of publication of application: **18.03.2026**
(21) Application number: 24200857.1
(22) Date of filing: 17.09.2024
(51) Int. Cl.: C09B 11/24, G01N 33/58

(54) **DYES WITH PH-DEPENDENT ABSORPTION AND FLUORESCENCE**

(71) Applicant: ATTO-TEC Gmbh, 57074 Siegen (DE)
(72) Inventor: SYAM, Aswathi, 57074 Siegen (DE); ZILLES, Alexander, 57223 Kreuztal (DE); KEMNITZER, Norbert, 51643 Gummersbach (DE); ARDEN-JACOB, Jutta, 90513 Zimdorf (DE)
(74) Representative: Maiwald GmbH

(57) **Abstract**

The present invention relates to dyes with pH-dependent absorption and fluorescence for pH detection and their applications in biology, medicine and theranostic.

In particular, the present invention relates to compounds of the general formulae (I) - (IV), that show pH dependent absorption and fluorescence spectra as well as pH dependent fluorescence quantum yield, methods for their preparation and their application as pH-sensors and labeling groups for the detection of analytes.

## Description

The present invention relates to dyes with pH-dependent absorption and fluorescence for pH detection and their applications in biology, medicine and theranostic.

In particular, the present invention relates to compounds of the general formulae (I) - (IV), that show pH dependent absorption and fluorescence spectra as well as pH dependent fluorescence quantum yield, methods for their preparation and their application as pH-sensors and labeling groups for the detection of analytes.

### Background of the invention

The pH plays a major role in many areas of daily life, e.g. shelf life or quality of food, skin protection barrier, cleaning agents etc. The pH is traditionally measured either electrochemically with a pH electrode or optically, i.e. usually via a color change as with classic indicators, e.g. litmus, phenolphthalein, etc.

However, the pH can also be determined using fluorescence. For example, fluorescent dyes whose fluorescence intensity or fluorescence wavelength depends on the pH can be used. Optical measurement via fluorescence is often even more accurate, as emitted light is measured more sensitively than absorption. This is particularly important when measuring in biological samples, where either a lower concentration or amount of pH sensor is required, or an early response is needed.

The pH can therefore also be used to monitor the healing process of a wound, as described in more detail in a) S.L. Percival, S. McCarty, J.A. Hunt, E.J. Woods: The effects of pH on wound healing, biofilms, and antimicrobial efficacy. Wound Rep. Reg. 22, 174 (2014); b) C. Waters, T. Yuan, K. Rumbaugh: Beneficial and deleterious bacterial-host interactions in chronic wounds pathophysiology. Chronic Wound Care Management and Research, 2, 52 (2015); or c) Z. Han, M. Yuan, L. Liu, K. Zhang, B. Zhao, B. He, Y. Liang, F. Li: pH-Responsive wound dressings; advances and prospects, Nanoscale Horizons, 8, 422 (2023).

When acute wounds occur, internal tissue and interstitial fluid with a pH of approximately 7.4 is exposed, changing the acidic milieu of normal skin, and the pH will return to acidic during the wound healing. Typically, the pH of a wound therefore drops during the healing process.

If the pH rises again at a later stage and remains at that elevated level, this strongly indicates an infected or chronic wound. Chronic wounds have a pH between 7 and 9 because of the existence of e.g. blood, interstitial fluid, ammonia. Most pathogenic bacteria increase the pH due to the metabolites generated by bacterial growth. However, there are also some pathogenic bacteria whose acidic metabolic products such as lactic acid and carbonic acid lead to a decrease of the pH in a wound. Therefore, pH is considered a reliable indicator of the wound status, and the variation in pH predicts a tendency for wound healing, infection and deterioration.

Hospital-acquired infections, also known as "nosocomial infections", and chronic infections in immunocompromised, immunosuppressed, or diabetic patients are a major global problem. Such complications lead to higher mortality rates and cause immense costs for the healthcare system. The early diagnosis of a pathogenic infection and its differentiation from a harmless sub-clinical colonization with bacteria would have several advantages, as a reduced, preventative use of antibiotics lowering the associated risk of resistance development, the unnecessary changes of dressings, associated with the risk of re-infection of the wound, and a general reduction of costs for the healthcare system.

There is therefore an urgent need for new strategies to detect and treat such wound infections. Standard bacterial cultures and testing are slow (up to 48 hours) and take place in laboratories away from the patient. In contrast, a pH-dependent Point-of-Care method would enable an immediate diagnosis through the real-time display of the pH change within a wound dressing - even with the additional possibility of triggering the release of drugs or antibiotics. A simple approach of an infection detecting wound dressing via pH monitoring is described in G. Panzarasa, A. Osypova, C. Toncelli, M.T. Buhmann, M. Rottmar, Q. Ren et al.: The pyranine-benzalkonium ion pair. A promising fluorescent system for the ratiometric detection of wound pH. Sensors and Actuators B: Chemical, 249, 156-160 (2017).

To summarize, it is important to maintain continuous and long-term monitoring of pH in chronic wounds, as pH is constantly changing during the healing process.

The so-called SNARF ("seminaphthorhodafluors") dyes were developed as pH indicators for the purpose of measuring intracellular pH, see US4945171A or O. Seksek, N. Henry-Toulme, F. Sureau, J. Bolard: SNARF-1 as an intracellular pH indicator in laser microspectrofluorometry. A critical assessment. Analytical Biochemistry, 1991, 193 (1), 49-54 (1991). Compared to the historically used fluorescein derivatives, these dyes were characterized by a higher photostability and a pH-dependent band shape or wavelength of the fluorescence spectrum. This enabled ratiometric measurements using the much more sensitive fluorescence spectroscopy to determine the intracellular pH. For example, both the acidic and basic form of the dye are excited at a particular wavelength simultaneously while the ratio of the two fluorescence intensities is correlated with the pH of the medium. The p*Kₐ* of SNARF dyes is in the range of 7.5 - 7.9 and therefore too high for the affordable detection of wound infections.

An object of the present invention is therefore to provide suitable fluorescent dyes that can be used as a pH sensor and/or as a marker group for the detection of the pH in analyte detection procedures, which in particular have absorption maxima which allow the use of inexpensive light sources, absorb outside the absorption range of natural occurring substances contained in biological samples, exhibit good solubility, high photostability or/and are characterized by high fluorescence quantum yield and/or a high turn-on-ratio, in order to at least partially avoid the disadvantages of prior art. In particular, it was an object of the present invention to provide small organic dye molecules suitable for colorimetric or ratiometric measurements using the different absorption and fluorescence wavelengths and/or fluorescence quantum yield of the acidic and basic form and having a p*Kₐ* within the range of 4 to 7.

### Summary of the invention

This object is achieved by compounds of the general formulae (I) - (IV) and their use as a sensor dye or marker group in a procedure for detecting analytes.

The present invention relates to a dye having pH-dependent absorption and/or pH-dependent fluorescence having general formula (I), (II), (III) or (IV) 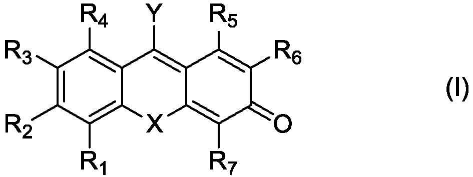 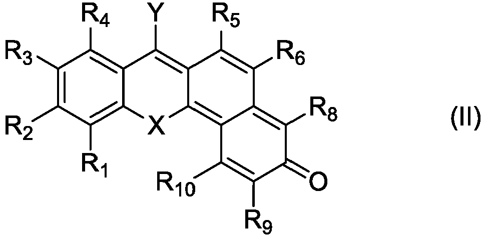 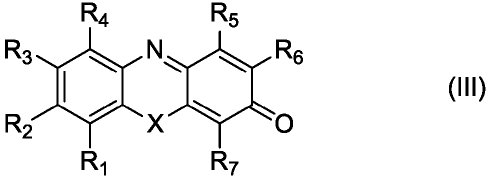 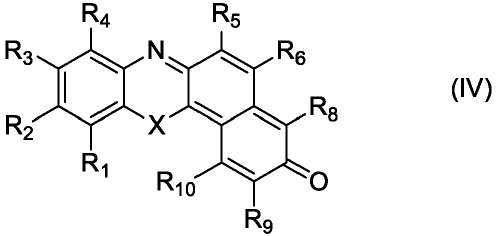 wherein
X = CR₁₁R₁₂, SiR₁₃R₁₄, sulfur, SO₂ or P(O)OR₁₅,
Y = hydrogen, a nitril group, a carboxyl group, a carboxylic acid derivative, an amino group or a straight-chain, branched or cyclic, saturated or unsaturated hydrocarbon moiety having up to 40 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
R₁, R₃, R₄, R₅, R₆, R₇, R₈, R₉, R₁₀ independently are hydrogen, halogen, a hydroxyl group, thiol group, amino group, sulfo group, phospho group, nitro group, carbonyl group, carboxyl group, a carboxylic acid derivative, a nitrile group, isonitrile group, cyanate group, thiocyanate group, isothiocyanate group or a straight-chain, branched or cyclic saturated or unsaturated hydrocarbon moiety having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
R₁₁, R₁₂, R₁₃, R₁₄, R₁₅, R₃₁, R₃₂ independently are hydrogen or a hydrocarbon group having 1-20 carbon atoms, wherein the hydrocarbon group optionally comprises one or more heteroatoms selected from the group of N, O, and S, or/and one or more substituents preferably selected from the group consisting of halogen, nitrile group, hydroxyl group, thiol group, amino group, nitro group, sulfo group, phospho group, carbonyl group, carboxyl group, a carboxylic acid derivative (such as carboxylate, ester, halide, amide, anhydride),
or R₁₁ and R₁₂ or R₁₃ and R₁₄, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SO₃H, CONR₃₁R₃₂ and a hydrocarbon group having from 1 - 20 carbon atoms or/and can be fused with one or more 3- to 7-membered rings, wherein the hydrocarbon group optionally comprises one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SO₃H, or CONR₃₁R₃₂;
R₂ = NR₁₆R₁₇ or R₁ together with R₂ is 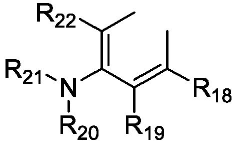
in which R₁₈, R₁₉ and R₂₂ are defined as R₁ and
R₁₆, R₁₇, R₂₀, R₂₁ are defined as R₁₁
or wherein at least one of R₁-R₂₂ forms a ring system with one or more neighboring moieties.

Preferred embodiments of the inventive dye having pH-dependent absorption and/or pH-dependent fluorescence are those given herein for the inventive compounds.

The inventive dyes have pH-dependent absorption and/or emission properties.

In particular, the inventive dyes have pH associated absorption properties.

Preferably, the inventive dyes show a color change in absorption depending on the pH.

The shift in wavelength associated with the color change is at least more than 50 nm, preferably more than 75 nm, which allows a good distinction of the color change. This color change occurs between the acid and basic form and is therefore associated with the p*Kₐ* of the dye.

Preferably, the inventive dyes have a p*Kₐ* in a range of pH 3 - pH 8, more preferably a p*Kₐ* in a range of pH 4 - pH 7 and most preferably a p*Kₐ* in a range of pH 5.5 - pH 6.5. The inventive dyes having a p*Kₐ* in this range are particularly useful for physiological measurements and, most preferably in detection of wound infections.

The inventive dyes in particular have an absorption maximum in the wavelength range of from 500 nm to 800 nm, in particular from 600 nm to 700 nm. Thus, for exciting the compounds and/or dyes inexpensive light sources, such as diode lasers can be used. Further, such absorption maxima allow an excitation outside the absorption range of naturally occurring substances contained in biological samples. This enables a more sensitive detection of the emission or fluorescence due to a low background emission from those biological substances.

The inventive dyes further preferably have a pH-depending emission. In a preferred embodiment, the inventive dyes show an emission of different characteristic wavelengths depending on the pH of the surrounding medium facilitating either the acidic or basic form of the dye. Preferably, the shift in emission wavelength between the acidic and basic form is at least more than 5 nm, more preferably more than 10 nm.

The inventive compounds and dyes, respectively, further preferably show a fluorescence quantum yield of at least 2% in their basic form, more preferably of at least 10% and even more preferably of at least 30%.

Further, the inventive dyes preferably show a change in emission or fluorescence intensity depending on the pH of the surrounding medium. Thus, the inventive dyes can in particular be used as a fluorogenic or a ratiometric sensor. In particular, the inventive dyes show a high turn-on-ratio of at least 2, i.e. the fluorescence of the basic form is at least 2 times higher than the fluorescence of the acidic form. More preferably the turn-on-ratio is at least 10, even more preferably at least 25 and up to 50.

The inventive dyes preferably have a Stokes-shift, i.e. a wavelength difference of absorption and fluorescence maximum, of at least 30 nm, more preferably at least 50 nm and most preferably at least 60 nm and up to 130 nm, preferably up to 120 nm and most preferably up to 110 nm.

The inventive dyes, further show a high photostability, comparable high as the photostablitity of the most photostable organic dyes and chromophors derived from the xanthene system. Commercially available examples thereof on the market are e.g. Rhodamine 6G, ATTO 647N and ATTO 643. All of them are well known for their very high photostability.

The inventive compounds and dyes, respectively, further exhibit a distinct solvatochromism, i.e. their absorption and fluorescence spectra are influenced by the solvent or surrounding medium. This photophysical property can be useful for the investigation and interpretation of solvent parameters, e.g. polarity, H-bond donor or acceptor characteristics, ionic strength, permittivity.

The very photostable inventive dyes can be used as a conventional indicator dye due to the pH associated color change in absorption. Beside that, the dyes have emission of different characteristic wavelength dependent on the pH of the surrounding medium and thus can be used as a fluorogenic or ratiometric sensor. Furthermore, the dyes can also be used as a sensor for solvent polarity as they show a distinct solvatochromism.

The dyes are therefore suitable for many applications as a pH sensor, especially for biological or medical purposes. Dyes showing a p*Kₐ* near pH 6, e.g. can be used as a sensor for bacterial growth within smart wound dressings for the determination of chronic wound infections or in bacterial biofilms.

The present invention further relates to a compound having general formula (I), (II), (III) or (IV) 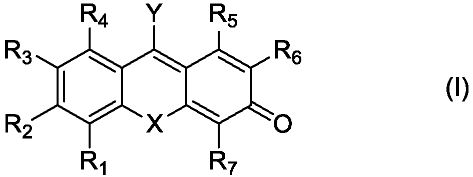 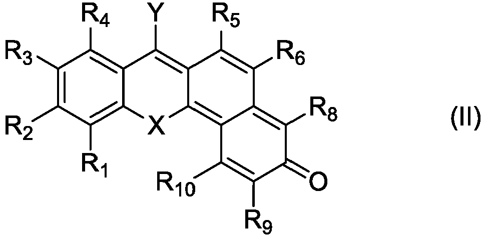 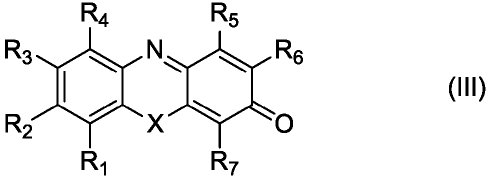 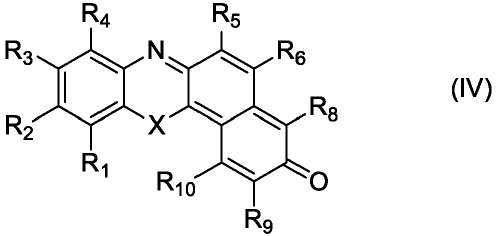 wherein
X = CR₁₁R₁₂, SiR₁₃R₁₄, sulfur, SO₂ or P(O)OR₁₅,
Y = hydrogen, a nitril group, a carboxyl group, a carboxylic acid derivative (such as carboxylate, ester, halide, amide, anhydride), an amino group or a straight-chain, branched or cyclic, saturated or unsaturated hydrocarbon moiety (such as alkyl, cycloalkyl, aryl, heteroaryl) having up to 40 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
R₁, R₃, R₄, R₅, R₆, R₇, R₈, R₉, R₁₀ independently are hydrogen, halogen, a hydroxyl group, thiol group, amino group, sulfo group, phospho group, nitro group, carbonyl group, carboxyl group, a carboxylic acid derivative (such as carboxylate, ester, halide, amide, anhydride), a nitrile group, isonitrile group, cyanate group, thiocyanate group, isothiocyanate group or a straight-chain, branched or cyclic saturated or unsaturated hydrocarbon moiety (such as alkyl, cycloalkyl, aryl, heteroaryl) having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
R₁₁, R₁₂, R₁₃, R₁₄, R₁₅, R₃₁, R₃₂ independently are hydrogen or a hydrocarbon group having 1-20 carbon atoms, wherein the hydrocarbon group optionally comprises one or more heteroatoms selected from the group of N, O, and S, or/and one or more substituents preferably selected from the group consisting of halogen, nitrile group, hydroxyl group, thiol group, amino group, nitro group, sulfo group, phospho group, carbonyl group, carboxyl group, a carboxylic acid derivative (such as carboxylate, ester, halide, amide, anhydride),
or R₁₁ and R₁₂ or R₁₃ and R₁₄, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, CONR₃₁R₃₂ and a hydrocarbon group having from 1 - 20 carbon atoms or/and can be fused with one or more 3- to 7-membered rings, wherein the hydrocarbon group optionally comprises one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SO₃H, or CONR₃₁R₃₂;
R₂ = NR₁₆R₁₇ or R₁ together with R₂ is 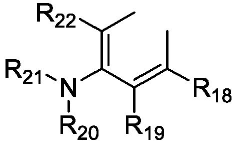
in which R₁₈, R₁₉ and R₂₂ are defined as R₁ and
R₁₆, R₁₇, R₂₀, R₂₁ are defined as R₁₁
or wherein at least one of R₁-R₂₂ forms a ring system with one or more neighboring moieties,
   with the proviso,
   that in a compound of formula (I) there are not simultaneous:
      X = C(CH₃)₂;
      R₁, R₃, R₄, R₅, R₆ and R₇ = H,
      R₂ = N(CH₃)₂; and
      Y = 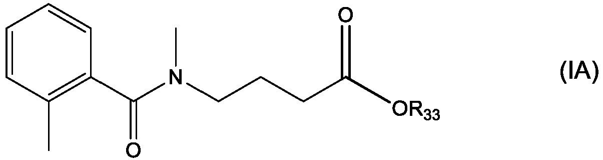
      wherein R₃₃ = H or CH_{3;} and
      that in a compound of formula (I) there are not simultaneous
      X = C(CH₃)₂;
      R₁, R₃, R₄, R₅, R₆ and R₇ = H
      R₂ = N(CH₃)₂; and
      Y = 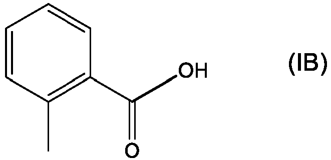 and
      that in a compound of formula (I) there are not simultaneous X = C(CH₃)₂;
      R₁, R₃, R₄, R₅, R₆ and R₇ = H
      R₂ = N(CH₃)₂; and
      Y = 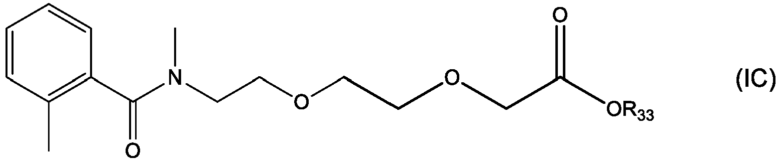
      wherein R₃₃ = H or CH_{3;} and
      that in a compound of formula (I) there are not simultaneous
      X = Si(CH₃)₂;
      R₁, R₃, R₄, R₅, R₆ and R₇ = H
      R₂ = NH₂, NH(CH₃), N(CH₃)₂ or NH-C(O)CH₃; and
      Y = 1-methyl-phenyl,
      and wherein compounds of formula (III) with X = CR₁₁R₁₂ comprise at least one sulfo group.

The term "hydrocarbon group", as used in the scope of the present application, comprises a saturated or unsaturated hydrocarbon moiety having 1-40 carbon atoms, preferably 1-20 carbon atoms, more preferably 1-12 carbon atoms, which can be linear, branched, or cyclic and has a bond valence at any one of the carbon atoms.

Examples of hydrocarbon groups according to the present invention include alkyl, cycloalkyl, alkenyl or alkynyl and aryl.

The hydrocarbon group may comprise one or more heteroatoms selected from the group consisting of N, O and S. Thus, a hydrocarbon group can also be a heteroalkyl, heterocycloalkyl, or heteroaryl group. A hydrocarbon group may comprise one or more substituents, preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, and CON(alkyl)₂.

The term "aliphatic hydrocarbon group", as used in the scope of the present application, has the same meaning as hydrocarbon group, except for aryl or heteroaryl.

The term "alkyl", as used in the scope of the present application, means a saturated, linear, or branched hydrocarbon moiety having 1-40 carbon atoms, preferably 1-20 carbon atoms, more preferably 1-12 carbon atoms, which has a bond valence at any one of the carbon atoms. Preferably alkyl constitutes a hydrocarbon moiety having 1-12 carbon atoms, more preferably having 1-6 carbon atoms. Especially preferable alkyl comprises methyl, ethyl, n-propyl, iso-propyl, n-butyl, iso-butyl, sec-butyl and tert-butyl.

If the hydrocarbon moiety comprises one or more heteroatoms selected from the group consisting of N, O and S, the moiety can be a heteroalkyl and thus comprise inter alia alkoxy groups such as methoxy, ethoxy etc.

The term "cycloalkyl", as used in the scope of the present application, means a saturated or unsaturated, cyclic hydrocarbon moiety having 3-20 carbon atoms, which has a bond valence at any one of the 3-20 carbon atoms. Preferably cycloalkyl constitutes a cyclic hydrocarbon moiety having 3-12 carbon atoms, more preferably having 3-8 carbon atoms. Especially preferable cycloalkyls comprise cyclopropyl, cyclobutyl, cyclopentyl, cyclohexyl, cycloheptyl, and cyclooctyl. In addition the cyclic hydrocarbon moieties can be interconnected and form bicyclic or polycyclic compounds, such as norbornane, norbornene, bicyclo[2.2.2]octyl, bicyclo[3.2.2]nonyl, and adamantyl.

If the cyclic hydrocarbon moiety comprises one or more heteroatoms selected from the group consisting of N, O and S, the moiety can also be a heterocycloalkyl, and thus comprise aliphatic heterocycles such as tetrahydropyrrole, piperidine, dioxane, or tetrahydrofuran.

The term "alkenyl", as used in the scope of the present application, means an unsaturated, linear, or branched hydrocarbon moiety having 2-20 carbon atoms, which has a bond valence at any one of the 2-20 carbon atoms and at least one double bond. Preferably alkenyl constitutes a hydrocarbon moiety having 2-12 carbon atoms, more preferably having 2-6 carbon atoms. Especially preferable alkenyls comprise ethenyl, propenyl and butenyl.

The term "alkynyl", as used in the scope of the present application, means an unsaturated, linear, or branched hydrocarbon moiety having 2-20 carbon atoms, which has a bond valence at any one of the 2-20 carbon atoms and at least one triple bond. Preferably alkynyl constitutes a hydrocarbon moiety having 2-12 carbon atoms, more preferably having 2-6 carbon atoms. Especially preferable alkynyls comprise ethynyl, propynyl and butynyl.

The term "aryl", as used in the scope of the present application, means an aromatic ring system having 3-20 ring atoms, more preferably having 6-14 ring atoms, which contains only carbon atoms and has a bond valence at any one of the carbon atoms of the 3-20 ring-forming atoms. Preferable aryls comprise phenyl, naphthyl, anthracenyl, phenanthrenyl, pyrenyl.

The term "heteroaryl", as used in the scope of the present application, means an aromatic ring system having 3-20 ring atoms, more preferably having 5-14 ring atoms, which as ring atoms, apart from carbon atoms contains at least one heteroatom selected from the group consisting of N, O and S and has a bond valence at any one of the carbon atoms or nitrogen atoms of the 3-20 ring-forming atoms. Preferable heteroaryls comprise furanyl, imidazolyl, isothiazolyl, isoxazolyl, oxadiazolyl, oxazolyl, pyrazolyl, pyrrolyl, tetrazolyl, thiadiazolyl, thiazolyl, thienyl, triazolyl, pyrazinyl, pyridazinyl, pyridyl, pyrimidinyl, and triazinyl.

The term "halogen", as used in the scope of the present application, comprises fluorine, chlorine, bromine, and iodine, in particular fluorine and chlorine.

The term "sulfo group" preferably designates a sulfonic acid group or a sulfuric acid group. Preferably, the term sulfo group designates an SOsH-group.

The term "phospho group" preferably designates a phosphonic acid group or a phosphoric acid group.

It is preferred according to the invention
that in a compound of formula (I) there are not simultaneous
X = C(CH₃)₂;
R₁, R₃, R₄, R₅, R₆ and R₇ = H,
R₂ = N(CH₃)₂; and
Y = 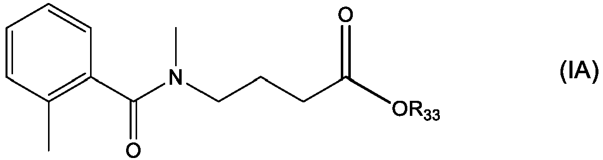
wherein R₃₃ = H or CH_{3;} and
that in a compound of formula (I) there are not simultaneous
X = C(CH₃)₂;
R₁, R₃, R₄, R₅, R₆ and R₇ = H
R₂ = N(CH₃)₂; and
Y = 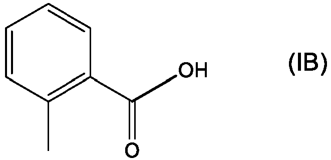 and
that in a compound of formula (I) there are not simultaneous
X = C(CH₃)₂;
R₁, R₃, R₄, R₅, R₆ and R₇ = H
R₂ = N(CH₃)₂; and
Y = 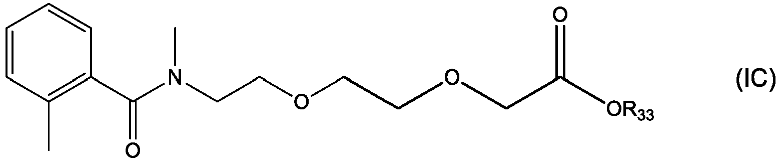
wherein R₃₃ = H or CH₃.

It is further preferred that in a compound of formula (I) there are not simultaneous
X = Si(CH₃)₂;
R₁, R₃, R₄, R₅, R₆ and R₇ = H
R₂ = NH₂, NH(CH₃), N(CH₃)₂ or NH-C(O)CH₃; and
Y = 1-methyl-phenyl.

In preferred embodiments of the invention R₁, R₃, R₄, R₅, R₆ and R₇ = H.

In further preferred embodiments at least one of R₁, R₃, R₄, R₅, R₆ and R₇ = a substituent containing a COOH group, a SOsH group, and/or a halogen, in particular Cl or F. More preferably R₃ is a substituent containing a COOH group, a SOsH group, in particular CH₂SO₃H and/or a halogen, in particular Cl or F.

R₂ is preferably R₂ = NR₁₆R₁₇, wherein R₁₆ and R₁₇ are C1 - C4 alkyl, most preferably R₁₆ = R₁₇ = methyl. Compounds in which R₂ = N(CH₃)₂ are preferred, since such compounds have a relatively simple structure and can be easily prepared.

Further preferred R₂ = NR₁₆R₁₇, wherein R₁₇ = C1 - C4 alkyl, most preferably R₁₇ = CH₂CH₃ and R₁₆ and R₃ together form a ring system having the formula:

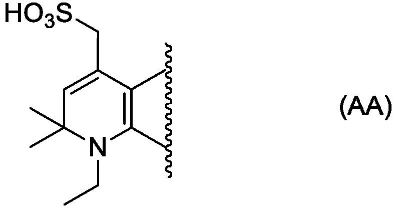

Further preferred R₂ = NR₁₆R₁₇, wherein R₁₇ = C1 - C4 alkyl, most preferably R₁₇ = CH₂CH₃ and R₃ and R₁₆ together form a ring system having the formula:

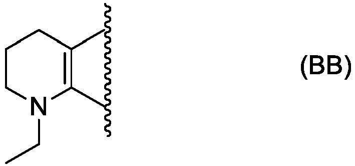

In a further preferred embodiment R₂ = NR₁₆R₁₇, wherein at least one of R₁₆ and R₁₇ is a hydrocarbon group having 1-20 carbon atoms, which comprises one or more hetero atoms selected from oxygen, in particular wherein at least one of R₁₆ and R₁₇ comprises a polyether, preferably a polyethylene glycol and/or a polypropylene glycol group having 2-20 carbon atoms. Such groups impart a good water solubility to the compounds without adding further negative charges.

In a further preferred embodiment, R₂ = NR₁₆R₁₇, wherein at least one of R₁₆ and R₁₇ is a long chain hydrocarbon group having at least 13 carbon atoms, more preferably at least 15 carbon atoms. Particularly preferred groups are C13 to C20 alkyl groups. Such groups impart a high hydrophobicity to the structures and those compounds can e.g. be embedded into polymers.

Each of the moieties present in the inventive compounds of formulae (I) - (IV) may optionally form a ring system with one or more neighboring moieties. Said ring systems preferably comprises 5- and/or 6-membered rings. Preference is given to such ring systems being formed by R₁ and R₁₆ and/or R₃ and R₁₇ resulting in the following structures (A), (B), (C), (D), (E), (F), and (G) 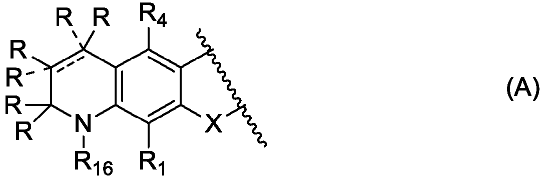 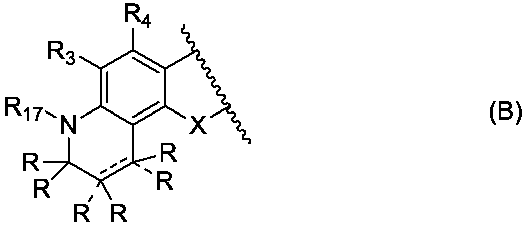 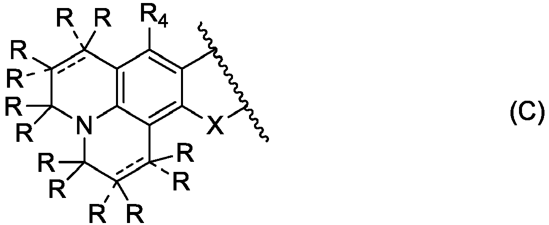 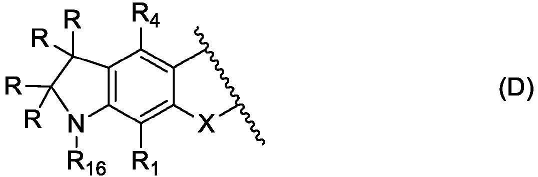 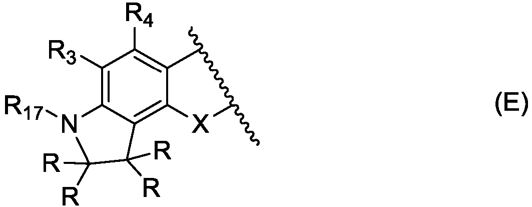 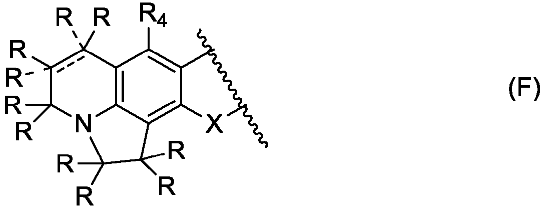 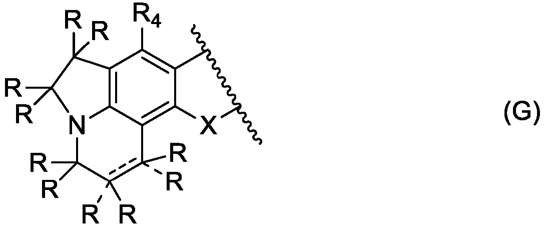 in which R in each case independently is defined as R₁ and the dashed lines are optionally double bonds in the presence of which the moieties bound via a dashed line are absent.

In the structures (A) - (G) neighboring substituents R may optionally form further ring systems which comprise 5- and/or 6-membered rings which may optionally contain further heteroatoms or substituents.

In the same way as shown in the structures (A) - (G), 5- and/or 6-membered rings can be formed by the moieties R₃ and R₁₇ and/or R₁ and R₁₆. The resulting structures are likewise preferred embodiments of the invention.

Examples for such structures with additional rings include inter alia:

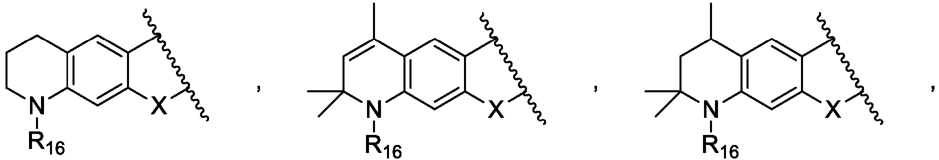

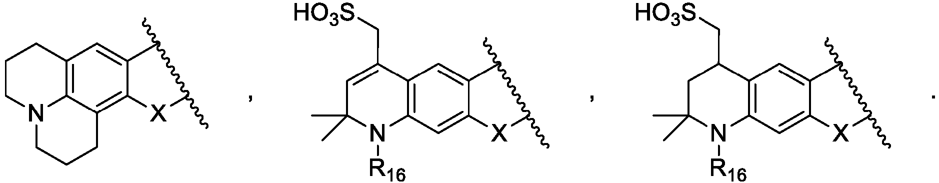

Structures having ring systems formed by R₁ and R₁₆ and/or R₃ and R₁₇, respectively, show a bathochromic shift in absorption and/or fluorescence of about 25 - 50 nm compared to the structures without such a ring system. This shift to longer wavelength is advantageous for various applications, because inexpensive light sources can be used and there is a lower background fluorescence due to the absence of natural occurring fluorophores enhancing the sensitivity of the detection.

Further preferred embodiments are structures with annulated ring systems. They preferably result from ring-formation by the moieties R₃ and R₄ and/or R₅ and R₆ and/or R₆ and R₈ and/or R₅ and R₆ and R₈ in formulae (I) - (IV). Preference is given to aromatic six-membered ring system as shown in the following examples:

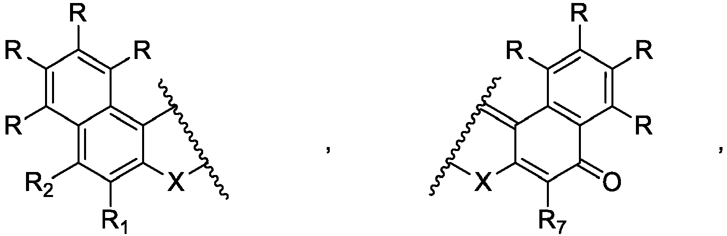

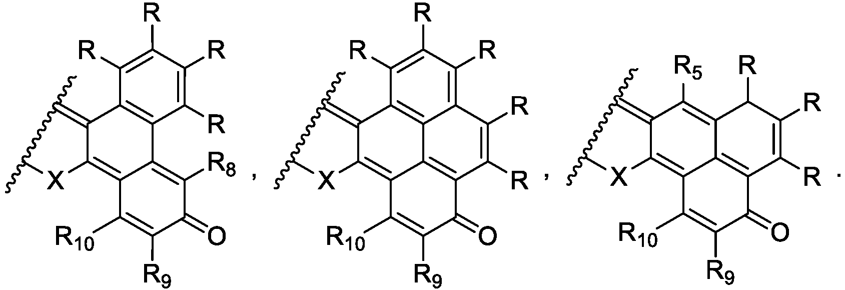

Positions R at each occurrence independently is defined as R₁.

According to the present invention, it was found that compounds of formula (I), (II), (III) or (IV) in which at least one group R₁ - R₂₁ forms a ring system with one or more neighboring moieties, whereby said ring system preferably comprises five- and/or six-membered rings have an absorption and/or fluorescence being at relatively long wavelength. I.e. those compounds show a shift into the visible and in particular into the red spectrum. Thus, the compounds have advantageous properties for many applications.

Further preferred according to the present invention Y in formulae (I) and (II) is optionally substituted cycloalkyl, optionally substituted phenyl, optionally substituted naphthyl or optionally substituted heteroaryl with the following structures (H), (J), (K), (L), (M), (N), and (O) 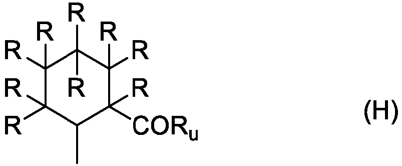 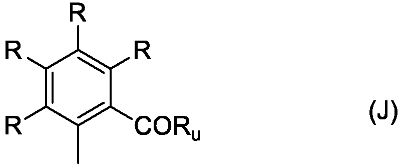 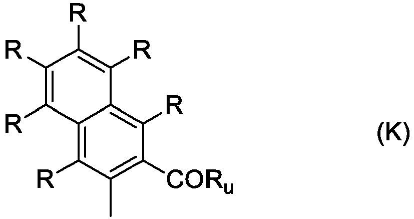 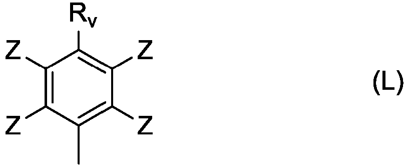 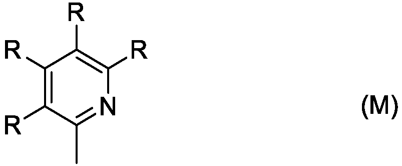 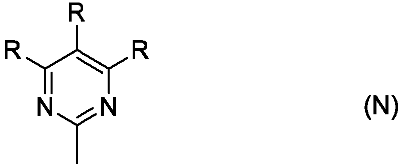 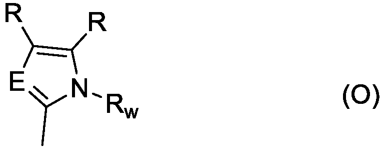 wherein
E = oxygen, sulfur, nitrogen or ⁺NR₂₃
Rᵤ = halogen, OR₂₄, NHR₂₅, NR₂₆R₂₇,
Z = hydrogen or halogen,
Rᵥ = Z, S(CH₂)ₓCOOH, S(CH₂)ₓSO₃H, NHR₂₈, NR₂₉R₃₀ and
R_{w} =hydrogen, alkyl, (CH₂)ₓCOOH, (CH2)ₓSO₃H, (CH₂)ₓOH, (CH₂)ₓSH, (CH₂)ₓNH₂, (CH₂)ₓZ with x = 1, 2, 3, 4, 5, 6,
R₂₃, R₂₄, R₂₅, R₂₆, R₂₇, R₂₈, R₂₉, R₃₀ are independently of one another a straight-chain, branched or cyclic, saturated or unsaturated hydrocarbon moiety (such as alkyl, cycloalkyl, aryl, heteroaryl) having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂ and SOsH, or CONR₃₁R₃₂,
and positions R at each occurrence independently are defined as R₁.

Particularly preferred Y is an aryl or heteroaryl group, having one or more substituents containing a COOH group, a SOsH group and/or a halogen, in particular Cl and/or F. More preferred Y is an aryl or heteroaryl group having one or more substituents selected from COOH; C(=O)-N(CH₃)-CH₂-CH₂-CH₂-COOH; SOsH; F and/or Cl.

COOH-groups are in particular suitable for coupling the inventive dyes, in particular for coupling the inventive dyes to a surface, to a carrier or to an analyte.

SOsH-groups are in particular suitable to enhance water solubility of the compounds and/or to add negative charges to the compound, which can e.g. be advantageous in electrophoretic applications for the discrimination of differently charged molecules without changing their optical properties.

Preferable groups Y include

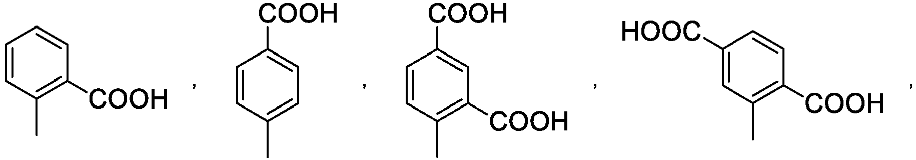

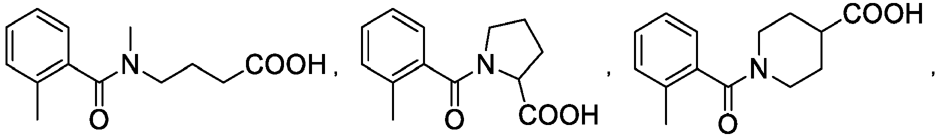

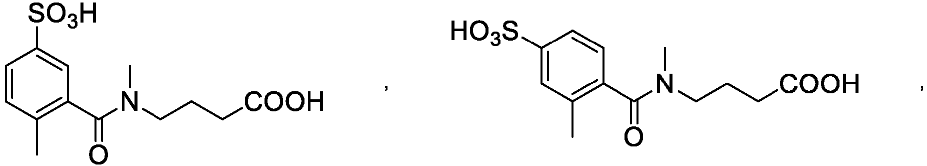

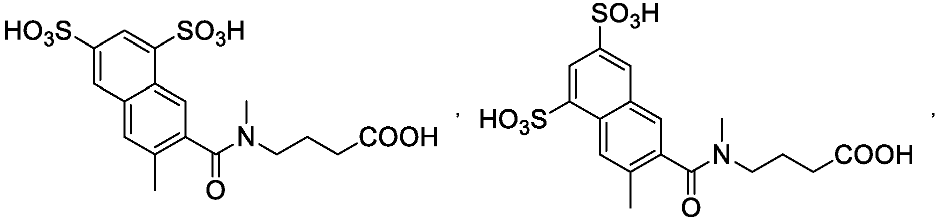

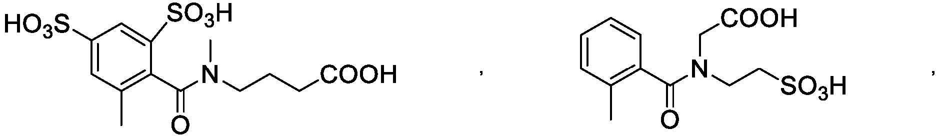

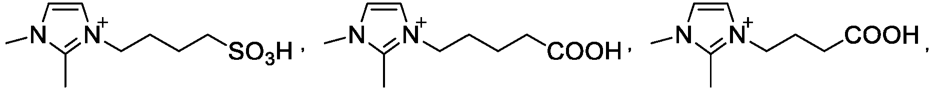

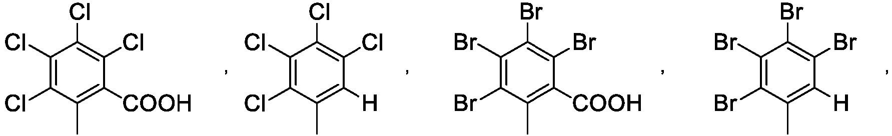

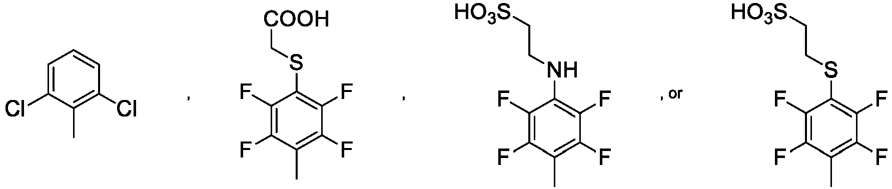

Particularly preferred are groups Y which comprise an ortho substituted aryl or heteroaryl group. It was found that such a substituent Y promotes chemical stability and colorfulness of the compounds in an alkaline pH range.

Further preferred are compounds of formula (I) and (II) in which
Y = 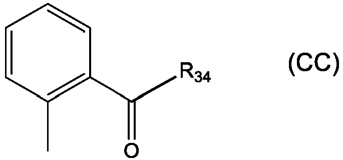 wherein R₃₄ = OH or N(CH₃)-(CH₂)ₓ-C(O)-OR₃₅ with x = 1 - 6 and R₃₅ = H or CH₃. In particular preferred is x = 3.

In particular preferred are residues Y comprising an imidazole group.

Further compounds having formula (I), (II), (III) or (IV) are preferred which have at least one halogen substituent, in particular at least one F and/or Cl substituent.

Further preferred, according to the present invention, are compounds which comprise at least one group SOsH group. Such compounds are in particular advantageous due to their good water solubility.

Further preferred are compounds of formula (I), (II), (III) or (IV) which comprise at least one carboxyl group (-COOH). Such carboxyl groups particularly can serve as linkage groups to attach the inventive compound covalently to a target molecule.

In a preferred embodiment of the present invention X in formulae (I) - (IV) is CR₁₁R₁₂ or SiR₁₃R₁₄. Even more preferred are the according structures where R₁₁ = R₁₂ = R₁₃ = R₁₄ is methyl, ethyl, propyl, or R₁₁ together with R₁₂ and/or R₁₃ together with R₁₄ form a four-, five-, or six-membered hydrocarbon ring system.

In a further preferred embodiment, R₁₁ = R₁₂ = R₁₃ = R₁₄ = phenyl, resulting in hydrophobic structures.

In a preferred embodiment of the present invention, the compound has general formula (I) or formula (II), more preferred the compound has formula (II).

X in such compounds of formula (I) or formula (II) is preferably CR₁₁R₁₂ or SiR₁₃R₁₄.

In a preferred embodiment X = CR₁₁R₁₂, such compounds are also termed carborhodol.

According to this preferred embodiment, R₁₁ and R₁₂ each independently are preferably a C1-C4 alkyl and most preferably R₁₁ = R₁₂ = CH₃.

Y in this preferred embodiment is preferably a phenyl group, or a imidazole group in particular substituted with one or more substituents selected from halogen, in particular Cl and/or F, C(O)OH, NH-CH₂-CH₂-SO₃H; S-CH₂-CH₂-SO₃H, C(O)-N(CH₃)-CH₂-CH₂-CH₂-C(O)OH, or C(O)-(1-piperidinyl)-4-carboxyl, or C(O)-(1-pyrrolidinyl)-2-carboxyl). Further, in particular preferred are Y substituents comprising at least one SOsH group.

In preferred embodiments of this embodiment R₁, R₃, R₄, R₅, R₆ and R₇ = H. In further preferred embodiments at least one of R₁, R₃, R₄, R₅, R₆ and R₇ = a substituent containing a COOH group, a SOsH group, and/or a halogen, in particular Cl or F. More preferably R₃ is a substituent containing a COOH group, a SOsH group, in particular CH₂SO₃H and/or a halogen, in particular Cl or F.

R₂ in this preferred embodiment is preferably R₂ = NR₁₆R₁₇, wherein R₁₆ and R₁₇ are C1 - C4 alkyl, most preferably R₁₆ = R₁₇ = methyl. Compounds in which R₂ = N(CH₃)₂ are preferred, since such compounds have a relatively simple structure and can be easily prepared. Further preferred R₂ = NR₁₆R₁₇, wherein R₁₇ = C1 - C4 alkyl, most preferably R₁₇ = CH₂CH₃ and R₁₆ and R₃ together form a ring system having the formula:

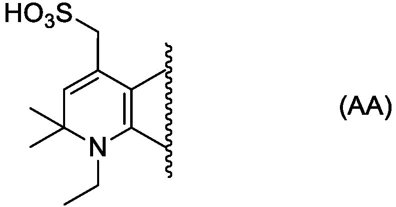

Further preferred R₂ = NR₁₆R₁₇, wherein R₁₇ = C1 - C4 alkyl, most preferably R₁₇ = CH₂CH₃ and R₃ and R₁₆ together form a ring system having the formula:

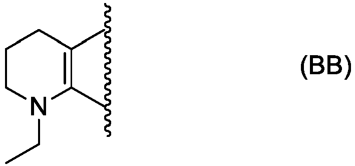

Further, compounds of formula (I) are preferred, wherein in a compound of formula (I) not simultaneous
X = C(CH₃)₂;
R₁, R₃, R₄, R₅, R₆ and R₇ = H,
R₂ = N(CH₃)₂; and
Y = 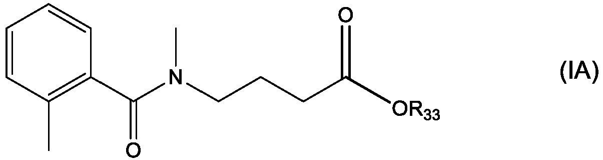
wherein R₃₃ = H or CH_{3;} and/or
wherein in a compound of formula (I) not simultaneous
X = C(CH₃)₂;
R₁, R₃, R₄, R₅, R₆ and R₇ = H
R₂ = N(CH₃)₂; and
Y = 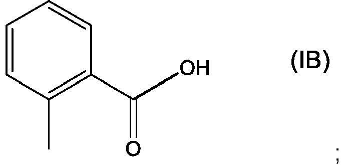 and/or
wherein in a compound of formula (I) not simultaneous
X = C(CH₃)₂;
R₁, R₃, R₄, R₅, R₆ and R₇ = H
R₂ = N(CH₃)₂; and
Y = 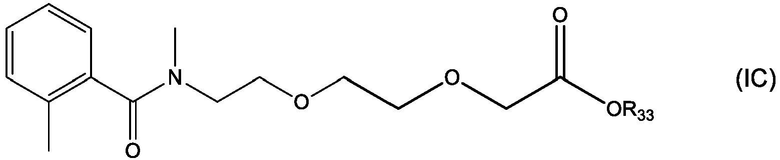
wherein R₃₃ = H or CH₃.

Further preferred are compounds of formula (I) in which Y is not 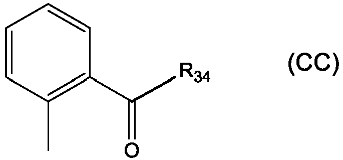 wherein R₃₄ = OH, C(O)-N(CH₃)-CH₂-CH₂-CH₂-C(O)-OR, with R = H or CH_{3;} or C(O)-N(CH₃)-CH₂-CH₂-O-CH₂-CH₂-O-CH₂-COOR₃₃, with R₃₃ = H or CH₃.

In another preferred embodiment, the inventive compound is of formula (I) or (II), wherein X = SiR₁₃R₁₄. Those compounds are also termed silico-rhodol. In those silico-rhodols R₁₃ and R₁₄ each independently are preferably a C1-C4 alkyl and most preferably R₁₁ = R₁₂ = CH₃.

In the inventive silico-rhodol Y preferably is a phenyl group, or a imidazole group in particular substituted with one or more substituents selected from halogen, in particular Cl and/or F, C(O)OH, NH-CH₂-CH₂-SO₃H; S-CH₂-CH₂-SO₃H, C(O)-N(CH₃)-CH₂-CH₂-CH₂-C(O)OH, or C(O)-(1-piperidinyl)-4-carboxyl, or C(O)-(1-pyrrolidinyl)-2-carboxyl. Further, in particular preferred are Y substituents comprising at least one SO₃H group.

Preferred are silico-rhodol compounds of formula (I), wherein Y is not 1-methyl-phenyl.

Further preferred are silico-rhodol compounds of formula (I), wherein in a compound of formula (I) not simultaneous
X = Si(CH₃)₂;
R₂ = NH₂, NH(CH₃), N(CH₃)₂ or NHC(O)CH₃; and
Y = 1-methyl-phenyl.

In yet a further embodiment, compounds having formula (III) or (IV), in particular formula (IV) are preferred.

Preferred structures of formulae (III) and (IV) include ring systems formed by R₁ and R₁₅ and/or R₃ and R₁₇. Examples of such preferable structures include

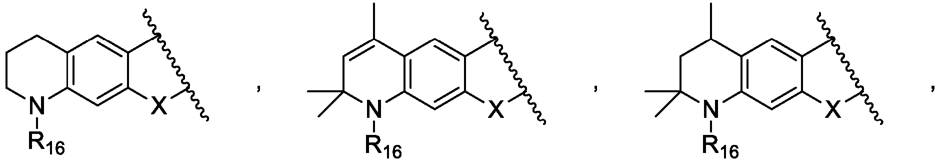

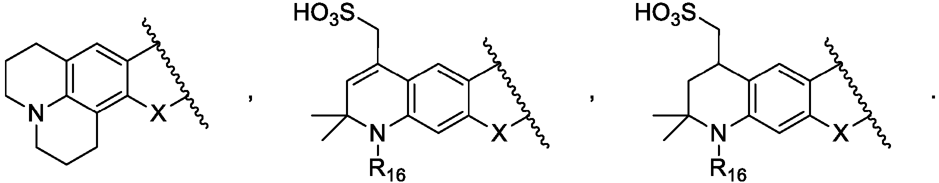

X in compounds of formula (III) or formula (IV) is preferably CR₁₁R₁₂ or SiR₁₃R₁₄.

If X = CR₁₁R₁₂, such compounds of formula (III) or (IV) are termed carbazines.

In particular preferred are such carbazines having at least one SOsH group and/or at least one COOH group.

Further preferred are carbazines in which R₂ = NR₁₆R₁₇ with R₁₆ = CH₃ and R₁₇ = CH₂-CH₂-CH₂-C(O)OH.

Further preferred are carbazines where R₆, R₇, R₈ and R₉ = H.

According to this preferred embodiment, R₁₁ and R₁₂ each independently are preferably a C1-C4 alkyl and most preferably R₁₁ = R₁₂ = CH₃. In a further preferred embodiment, R₁₁ = R₁₂ = phenyl, resulting in more hydrophobic structures.

In further preferred embodiments at least one of R₁, R₃, R₄, R₅, R₆, R₇, R₈, R₉, R₁₀ = a substituent containing a COOH group or a SOsH group. More preferably R₃ is a substituent containing a COOH group or a SOsH group, in particular CH₂SO₃H.

According to the invention, compounds of Formula (III), wherein X = CR₁₁R₁₂ comprise at least one sulfo group. Preferably, compounds of Formula (III), wherein X = CR₁₁R₁₂ comprise at least one sulfonic acid group. More preferably, in compounds of Formula (III), wherein X = CR₁₁R₁₂ at least one of R₁, R₃, R₄, R₅, R₆ or R₇ = SO₃H.

From the inventive compounds ionic/charged salts can be obtained. These salts may possess an altered solubility in polar, particularly aqueous media. Apart therefrom, such salts are equivalent to the shown formulae.

Furthermore, the compounds can in solution be present in a solvated, particularly hydrated form, or can contain one or more molecules of crystal water. Crystalline or amorphous forms are also equivalent to the shown formulae.

Some compounds may have asymmetric carbon atoms as optical active centers or double bonds. The racemates, diasteromers, and geometric isomers resulting therefrom are all covered by the present invention.

The compounds can further have unnatural isotope ratios or even be radioactively labelled. These isotope variants, whether or not they are radioactive, are also covered by the shown formulae.

The compounds according to the present invention are in particular suitable as dyes. Therefore, the present invention is also directed to a dye comprising a compound as defined herein.

It was surprisingly found that the inventive compounds have pH-dependent absorption and/or pH-dependent fluorescence. Therefore, in a preferred embodiment, the present invention is directed to a dye having a pH-dependent absorption and/or pH-dependent fluorescence.

The compounds of the general formulae (I) - (IV) can be used as a pH sensor, as their acidic and basic form have different absorption and fluorescence spectra as well as exhibit different fluorescence quantum yields. This is evidenced in more detail during the discussion of the properties of typical compounds of the inventive compounds.

The present invention therefore also relates to a pH sensor comprising an inventive compound and/or an inventive dye.

The detection or monitoring of the pH can either be done as a colorimetric measurement via absorption or as a ratiometric or fluorogenic measurement, making use of both absorption and fluorescence or of the different fluorescence quantum yield of the acidic and basic form of the compound and/or dye.

The compounds of the general formulae (I)-(IV) can further be used as marker groups in processes of qualitative or/and quantitative determination of an analyte.

For use as maker groups the inventive compounds preferably comprise a group which allows linkage of the compounds to an analyte, in particular a group which allows covalent linkage of the compound to an analyte. Therefore, preferred as marker groups are inventive compounds having at least one functional group capable of covalent coupling, such as OH, SH, NH₂, COOH, alkene, alkyne, azide, tetrazine for covalently linking the compound to an analyte.

The pH measurement in biological systems and fluids provides important information about the state of health, i.e. the functionality of cells, tissues, organs, or entire organisms. Intracellular pH changes are associated with endocytosis, calcium regulation, cell growth, chemotaxis, and cell adhesion. In individual cells or tissues, the pH is dependent on or correlates with external influences such as oxygen, light, ions, drugs, bacteria, or their metabolites.

The pH can therefore also provide information about the shelf life, quality and edibility of foods of different consistencies. Microbial processes triggered by bacteria, viruses or fungi and their metabolic products can lead to a change in the pH, which is associated with the degradation of the food.

The pH sensing or determination can preferably be made in aqueous fluids, e.g. samples of bodily fluids such as blood, serum, plasma, lymphatic fluid, bile, cerebrospinal fluid, extracellular tissue fluid, urine, saliva, and sweat, or wastewater or food. The method can be carried out both as a wet test, e.g. in a cuvette or as a dry test in corresponding reagent carrier. The carrier can be made of any material deemed suitable by a person skilled in the art that can be wetted by the sample to be examined. Examples of such carrier materials include porous glass, plastics, ion exchange resins, dextranes, celluloses, cellulose derivatives or/and hydrophilic polymers, but are not limited to these. The determination of the analyte can hereby be carried out by means of a single reaction or by a sequence of reactions. The analytes are frequently biomolecules, preferably selected from antibodies, nanobodies, enzymes, peptides, polypeptides, proteins, nucleotides, nucleosides, nucleic acids, nucleic acid analogs, or/and haptenes.

The compounds of the general formulae (I)-(IV) can therefore be used in all chemical, medical, and biological detection methods known to a person skilled in the art in which fluorescence dyes are suitable as marker groups. For this purpose, the compounds are generally covalently coupled to a receptor specific for the analyte to be identified, or to the analyte itself. Toward this end, the compounds according to the invention of the general formulae (I)-(IV) preferably have at least one functional group capable of covalent coupling, such as OH, SH, NH₂ and/or COOH, for example. One generally known process is coupling of the N-hydroxysuccinimidyl ester of the dye to an amino group of the substrate. The specific receptor or analyte can be any suitable compound or any suitable molecule, and is preferably a peptide, a polypeptide, or a nucleic acid. Numerous common coupling methods are described in Greg T. Hermanson, Bioconjugate Techniques, Academic Press, San Diego, Calif., 1996.

Apart from these conventional methods, in recent years the so-called "click-chemistry" has been established. Two molecules are joined together bio-orthogonally, i.e. the two reaction partners are reacted in a targeted manner in the presence of many other functionalities and without influencing the biological processes. Toward this end, the compounds according to the invention of the general formulae (I)-(IV) preferably have at least one functional group preferred in such "click reactions" such as azide, alkene, alkyne, or tetrazine. An overview of the principles may be found in K. Nwe, M. W. Brechbiel: Growing Applications of "Click Chemistry" for Bioconjugation in Contemporary Biomedical Research. Cancer Biotherapy and Radiopharmaceuticals, 24 (3), 289 (2009).

The inventive pH sensor can be designed and used for many applications, in particular for biological or medical purposes. The inventive pH sensor can in particular be used for detecting and/or determining bacterial growth.

The greatest sensitivity to small changes of the pH is achieved with an indicator if the p*Kₐ* of the substance is close to the pH of the medium under investigation. A p*Kₐ* range of pH 6 to pH 8 is therefore useful for physiological measurements. In detection of wound infections, a p*Kₐ* range of 5 - 7 is crucial.

In a preferred embodiment the present invention relates to a wound dressing comprising an inventive compound and/or an inventive dye and using the wound dressing for monitoring the condition or healing process of a wound.

This can be obtained by incorporating pH-sensitive dyes into wound dressings, which have the capacity to display the pH in real time by changing color or fluorescence. This avoids time-consuming laboratory tests or unnecessary re-dressings.

The use of the inventive compounds as a pH sensor in wound dressings include either the physical incorporation in self-assembled liposomes, polymersomes, vesicles, or capsules or the chemical coupling of the pH-sensitive dye to a carrier or support.

For the first case there are different mechanisms of pH sensing possible, e.g. the inventive compounds are trapped inside of such an ion-permeable vesicle and will respond to the pH of the surrounding wound medium in the known manner via a change in absorption and/or fluorescence. Another mechanism is the incorporation of pH responsive compounds at high concentration leading to fluorescence quenching. When the vesicle structure is opened or cleaved, e.g. by metabolites of pathogenic bacteria or other stimuli, the quenched molecules will be released leading to a lower concentration with an increase in fluorescence. Such constructs can be adjusted to different types of pathogens and combined with the simultaneous release of drugs, therapeutic and/or antibacterial agents. For the application in wound dressings these nanoscale reporter systems can themselves be incorporated or combined with hydrogels often used in commercial wound patches.

On the other hand, there can be a covalent bond between the pH responsive compound and the supporting material of the wound dressing. This leads to a strong chemical fixation of the compound and prevents an uncontrolled release into the wound area. Such supports in the dressing for chronic or complex wounds are often hydrogels based on chitin or chitosan, see the review H. Liu, C. Wang, C. Li, Y. Qin, Z. Wang, F. Yang, Z. Li, J. Wang: A functional chitosan-based hydrogel as a wound dressing and drug delivery system in the treatment of wound healing. RSC Advances 8, 7533 (2018). Other types or designs of carriers could be nanofibers, nanosheet bandage, composite films, nanoparticle clusters, microneedles, sponges, and foams.

Chitin and chitosan are generally considered to be biodegradable, biocompatible, non-antigenic, non-toxic, biologically adhesive, antimicrobial, biological activity, with a haemostatic effect, and are therefore ideal for the use in wound dressings. They can be combined with other natural or synthetic polymers, such as alginate, cellulose, glycosaminoglycans, poly(acrylate acid), poly(ethylene glycol), poly(ethylen oxide), poly(vinyl alcohol), poly(lactic acid) etc. to enhance the stability or permeability of the tissue.

The commercially available wound dressings of chitosan are available in the form of non-wovens, hydrogels, films, and sponges. These wound dressings are also able to deliver antibacterial agents, growth factors, stem cells, peptides and other active substances in a sustained release manner and therefore act as a theragnostic device.

The following examples in Table 1 and Table 2 serve to give a better understanding of the present invention but are not intended to limit the scope of the invention.

**Table 1: Compounds according to general formula (I) with X = CR₁₁R₁₂**

| (a): Acidic form; measurements in buffer pH 3 (Citric acid, NaOH and NaCl) | | | | | | | | |
|---|---|---|---|---|---|---|---|---|
| (b): Basic form; measurements in buffer pH 10 buffer (Boric acid, NaOH and KCl) | | | | | | | | |
| Compound | Structure | Turn on ratio = η_{fl} (b) / η_{fl} (a) | p*Kₐ* | λ_{abs} [nm] | λₑₘ [nm] | Stokes-shift [nm] | η_{fl} [%] | t_{fl}, [ns] |
| 1 | 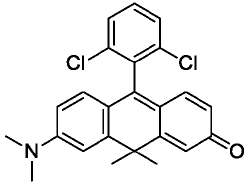 | 48 | 5.2 | 594 (b) | 618 (b) | 24 (b) | 35 (b) | 1.8 (b) |
| | | | | 518/551 (a) | 590 (a) | 72 (a) | 0.6 (a) | 0.2 (a) |
| 2 | 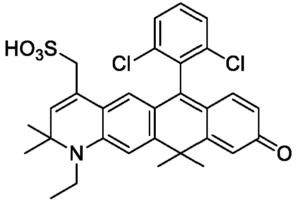 | 4 | 5.6 | 627 (b) | 658 (b) | 31 (b) | 15 (b) | 1.1 (b) |
| | | | | 545/581 (a) | 659 (a) | 114 (a) | 3 (a) | 0.5 (a) |
| 3 | 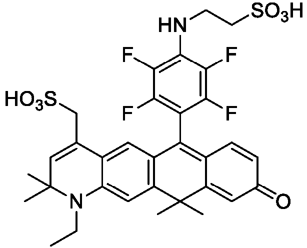 | 36 | 5.6 | 632 (b) | 662 (b) | 30 (b) | 8 (b) | 0.6 (b) |
| | | | | 548/591 (a) | 649 (a) | 101 (a) | 0.2 (a) | 0.3 (a) |
| 4 | 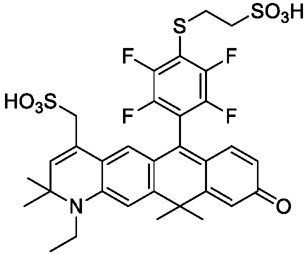 | 4 | 5.6 | 635 (b) | 669 (b) | 34 (b) | 6 (b) | 0.6 (b) |
| | | | | 547/590 (a) | 653 (a) | 106 (a) | 1.5 (a) | 0.2 (a) |
| 5 | 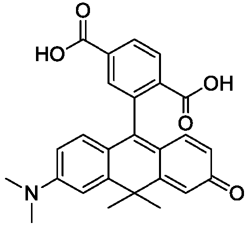 | 25 | 6.2 | 575 (b) | 605 (b) | 30 (b) | 32 (b) | 2.2 (b) |
| | | | | 510/546 (a) | 586 (a) | 76 (a) | 1.6 (a) | ---- |
| 6 | 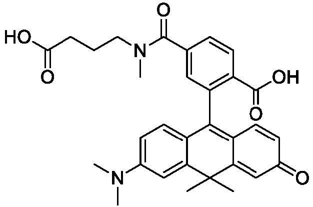 | 27 | 6.2 | 578 (b) | 605 (b) | 27 (b) | 43 (b) | 2.2 (b) |
| | | | | 512/547 (a) | 586 (a) | 74 (a) | 1.3 (a) | ---- |
| 7 | 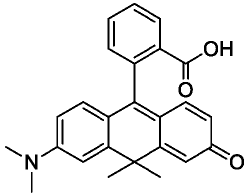 | 17 | 6.1 | 574 (b) | 602 (b) | 28 (b) | 35 (b) | 2.2 (b) |
| | | | | 510/546 (a) | 586 (a) | 76 (a) | 2 (a) | ---- |
| 8 | 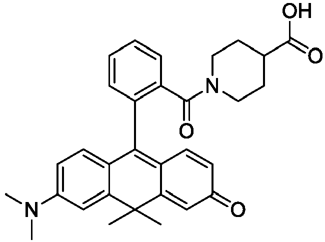 | 29 | 5.7 | 586 (b) | 614 (b) | 28 (b) | 36 (b) | 2.3 (b) |
| | | | | 516/551 (a) | 597 (a) | 81 (a) | 1.1 (a) | 0.2 (a) |
| 9 | 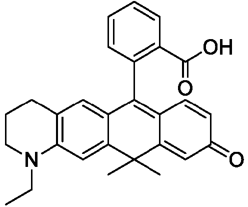 | 1.9 | 6.6 | 588 (b) | 614 (b) | 26 (b) | 49 (b) | 3(b) |
| | | | | 511/545 (a) | 597 (a) | 86 (a) | 22 (a) | 1.5 (a) |
| 10 | 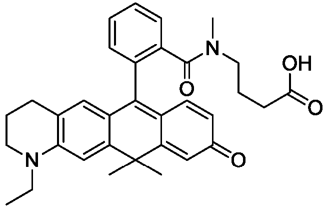 | 1.7 | 6.3 | 599 (b) | 624 (b) | 25 (b) | 43 (b) | 2.7 (b) |
| | | | | 515/551 (a) | 625 (a) | 110 (a) | 22 (a) | 1.6 (a) |

**Table 2: Compounds according to general formula (III) with X = CR₁₁R₁₂**

| (a): Acidic form; measurements in buffer pH 3 (Citric acid, NaOH and NaCl) | | | | | | | |
|---|---|---|---|---|---|---|---|
| (b): Basic form; measurements in buffer pH 10 buffer (Boric acid, NaOH and KCl) | | | | | | | |
| (c): Basic form; measurements in Ethanol | | | | | | | |
| Compound | Structure | Turn on ratio = η_{fl} (b) / η_{fl} (a) | p*Kₐ* | λ_{abs} [nm] | λₑₘ [nm] | Stokes-shift [nm] | η_{fl} [%] |
| 11 | 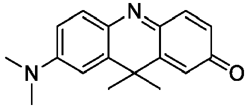 | 2.2 | 4.9 | 673 (b) | 694 (b) | 21 (b) | 2.1 (b) |
| | | | | 527 (a) | 653 (a) | 126 (a) | 0.95 (a) |
| | | | | 579 (c) | 675 (c) | 96 (c) | 11 (c) |
| 12 | 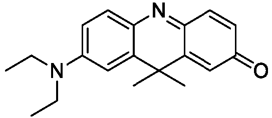 | ---- | ---- | ---- | ---- | ---- | ---- |
| | | | | ---- | ---- | ---- | ---- |
| | | | | 597 (c) | 688 (c) | 91 (c) | 15 (c) |
| 13 | 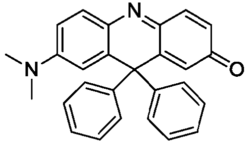 | ---- | ---- | ---- | ---- | ---- | ---- |
| | | | | ---- | ---- | ---- | ---- |
| | | | | 595 (c) | 708 (c) | 113 (c) | 5 (c) |
| 14 | 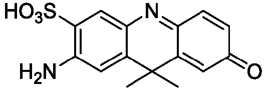 | ---- | ---- | 590 (b) | 650 (b) | 60 (b) | ---- |
| | | | | ---- | ---- | ---- | ---- |
| | | | | 558 (c) | 651 (c) | 93 (c) | 30 (c) |
| 15 | 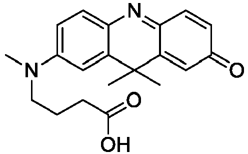 | ---- | ---- | 645 (b) | 695 (b) | 50 (b) | 4(b) |
| | | | | ---- | ---- | ---- | ---- |
| | | | | 600 (c) | 694 (c) | 94 (c) | 18 (c) |
| 16 | 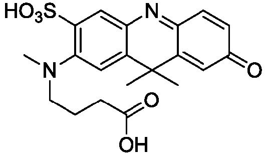 | ---- | ---- | ---- | ---- | ---- | ---- |
| | | | | ---- | ---- | ---- | ---- |
| | | | | 616 (c) | ---- | ---- | ---- |
| 17 | 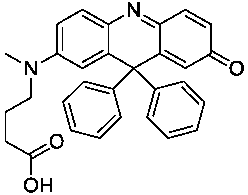 | 1.5 | 3.4 | 664 (b) | 719 (b) | 55 (b) | 1.2 (b) |
| | | | | 559 (a) | 712 (a) | 153 (a) | 0.8 (a) |
| | | | | 615 (c) | 721 (c) | 106 (c) | 6 (c) |
| 18 | 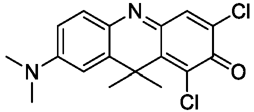 | ---- | ---- | 689 (b) | 755 (b) | 66 (b) | 4(b) |
| | | | | ---- | ---- | ---- | ---- |
| | | | | 630 (c) | 694 (c) | 64 (c) | 28 (c) |
| 19 | 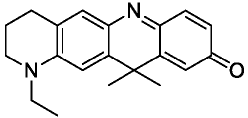 | ---- | ---- | ---- | ---- | ---- | ---- |
| | | | | ---- | ---- | ---- | ---- |
| | | | | 623 (c) | 694 (c) | 71 (c) | 12 (c) |
| 20 | 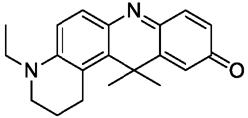 | ---- | ---- | ---- | ---- | ---- | ---- |
| | | | | ---- | ---- | ---- | ---- |
| | | | | 631 (c) | 697 (c) | 66 (c) | 10 (c) |
| 21 | 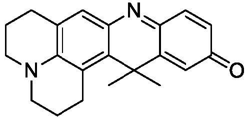 | ---- | ---- | ---- | ---- | ---- | ---- |
| | | | | ---- | ---- | ---- | ---- |
| | | | | 654 (c) | 704 (c) | 50 (c) | 15 (c) |

A subgroup of the compounds according to the present invention belong to the dye class of carborhodols, X = CR₁₁R₁₂ in general formulae (I) and (II). An example (compound 7) has been first described in M. Sednev, C. Wurm, V. Belov, S. Hell, Carborhodol: A Hybrid Fluorophore Obtained by Combination of Fluorescein and Carbopyronin Dye Cores. Bioconjugate Chemistry 24, 690 (2013). Despite mentioning the pH-equilibrium of those dyes - which is very similar to that of the long known rhodols - absolutely no reference is given to the use or suitability of such dyes as pH sensors or for ratiometric measurements. There is also no optical data available for the acidic form and the p*Kₐ* of the dye.

These compounds (see Table 1) do have several very interesting and advantageous properties compared to prior art, which make these compounds particularly suitable as ratiometric dyes and pH-sensors. This is shown below:

The measurement of the absorption spectrum at different pH clearly shows isosbestic points indicating the conversion between two defined species, i.e. the protonated (acidic) and deprotonated (basic) form, e.g. of compound 1:

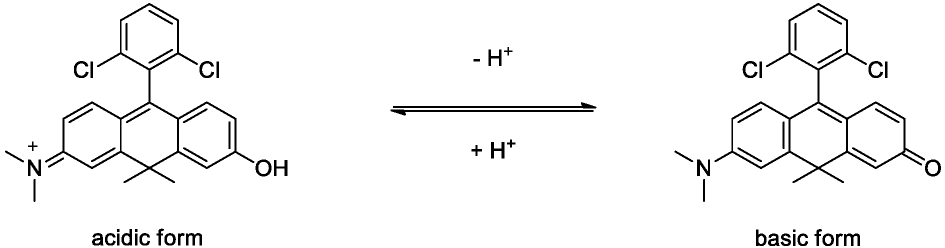

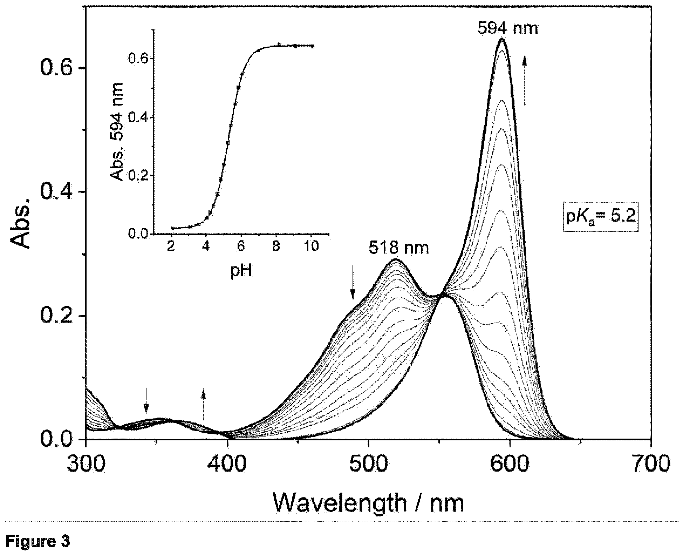

Due to the different absorption of both forms a color change according to the pH of the solution is visible with the naked eye. The compounds can therefore be easily used as pH sensors or indicator dyes for colorimetric measurements (see Figure 3).

Surprisingly and unexpectedly the compounds show a p*K*ₐ in the range of 5.5 - 6.5, (see Table 1), which is in stark contrast to the SNARF dyes. This perfectly enables the compounds according to the present invention as pH-sensors applied in wound dressings or for the reasonable and reliable detection of bacterial infections in chronic wounds or with pathological biofilms. Interestingly the p*K*ₐ is only very slightly influenced by changes of the molecular structure and the introduction of different substituents. This allows the structure to be freely adapted to desired optical and chemical properties without changing the critical p*K*ₐ significantly.

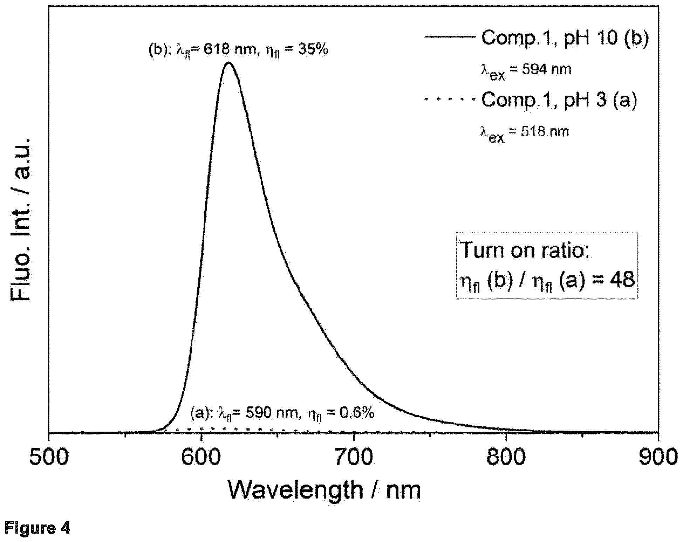

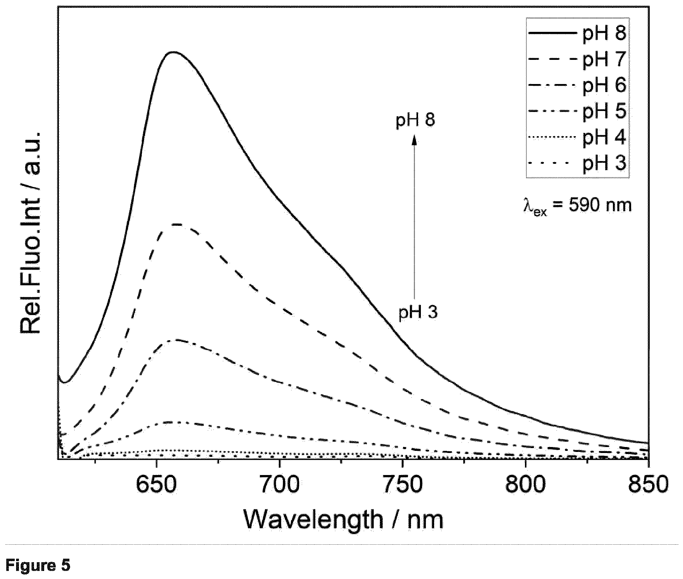

The basic form shows a much higher fluorescence than the acidic form, resulting in a "turn on"-ratio up to the factor 50 (see Table 1 and Figure 4). The increase of fluorescence intensity with increasing pH as the equilibrium is shifted towards the basic form is shown in Figure 5. Therefore, the compounds can be well used either as ratiometric or as fluorogenic sensors, e.g. to determine the pH of the surrounding medium or environment or its change during a chemical reaction, a biochemical or metabolic process etc.

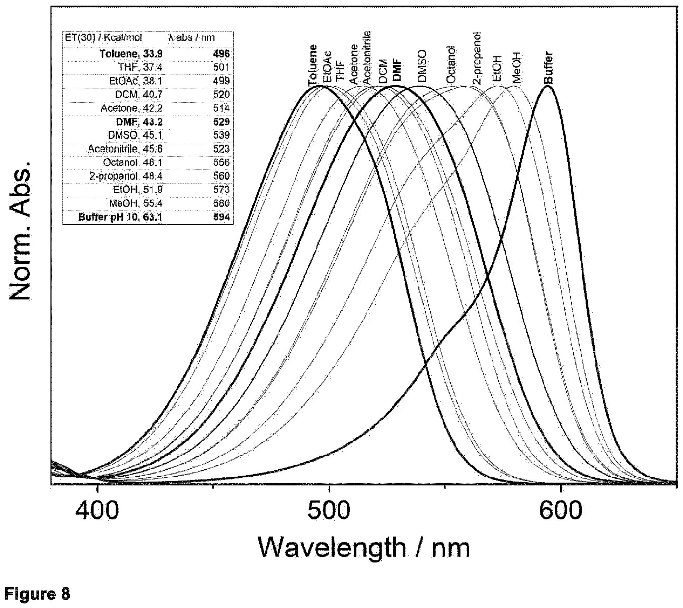

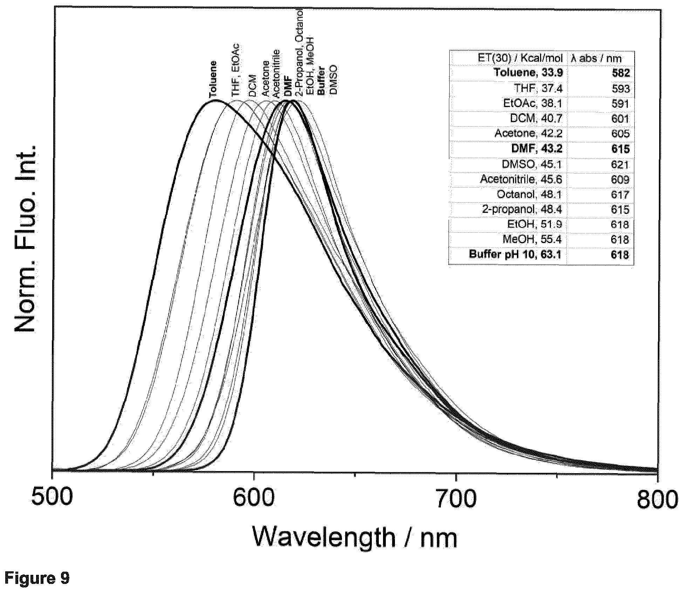

The compounds of the present invention are solvatochromic, i.e. the absorption and fluorescence maximum of the compounds is influenced by the solvent. Very similar to the well-known Reichardt's dye - defining the popular E_{T}(30)-scale of solvent polarity - the color of the solution changes depending on the solvent polarity (see C. Reichardt, Solvatochromic Dyes as Solvent Polarity Indicators, Chem. Rev. 94 (8), 2319 (1994)). Due to the bathochromic shift of the absorption maximum with increasing polarity this is called positive solvatochromism. This is shown for the basic dye form of compound 1 in a great variety of different solvents in Figure 8. Additionally, the fluorescence of the compounds is also dependent on the solvent polarity (see Figure 9). The basic form of the dye in the solvents other than aqueous buffer pH 10 was generated by the addition of Hünig's base.

An additional advantage of the compounds is their absorption and fluorescence at wavelengths above 550 nm or even above 600 nm in the red region of the visible spectrum. In this spectral region there is little inherent natural fluorescence in biological samples. Lower absorption and less scattering of light with increasing wavelength enable a greater depth of penetration of the excitation light into samples like blood and tissue. This facilitates a more reasonable and sensitive pH detection in biofilms or infected wounds, respectively. The absorption and fluorescence wavelengths of the compounds can be easily tuned by the introduction of different substituents like additional ring systems and/or double bonds. This can be seen by a comparison of compound 1 and compound 2 or compound 7 and compound 9 in Table 1.

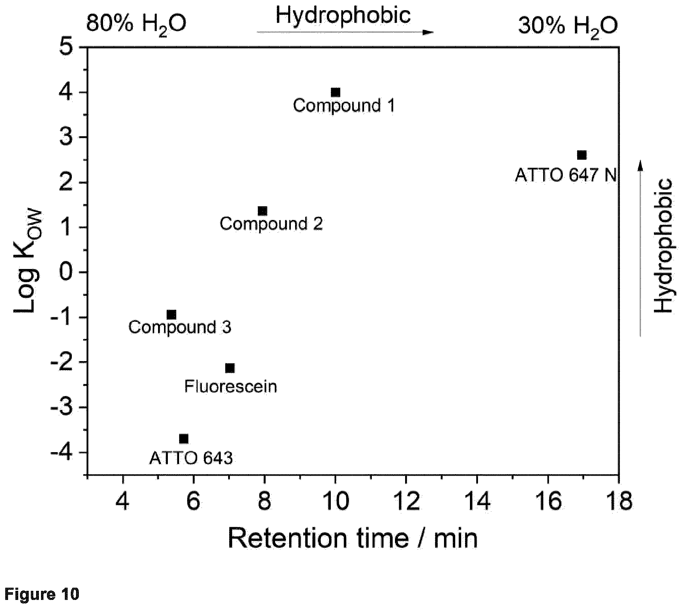

With the introduction of hydrophilic or hydrophobic groups it is also possible to change the solubility of the compounds. This is demonstrated by a comparison of compound 1 (without sulfonic acid group), compound 2 (with one sulfonic acid group), and compound 3 (with two sulfonic acid groups) with commercial dyes of known hydrophilicity. The result is that with the introduction of more sulfonic acid groups the inventive compounds become more hydrophilic and have a better solubility in water. This is verified by two different measurements (see Figure 10):
1) An increasing retention time in high performance reversed phase liquid chromatography corresponds to a higher hydrophobicity of a compound.
2) The partition coefficient between octanol and water, log(*K*_{ow}) is widely used as a measure of hydrophilicity in literature (see Y. Oba, S. R. Poulson: Octanol-water partition coefficients (Kow) vs. pH for fluorescent dye tracers (fluorescein, eosin Y), and implications for hydrologic tracer tests. Geochem. J. 46, 46, 517 (2012)). A lower (negative) value corresponds to hydrophilic compounds and a higher (positive) value indicates a hydrophobic compound.

The different solubility in water or polar solvents at the one hand and in nonpolar organic solvents on the other hand can be an important requirement for various types of applications, e.g. for the use as polymer-bound dye in smart wound dressings or when applied as a chemical sensor in vesicles. Due to the cleavage of the vesicle membrane by metabolic toxins of pathogenic bacteria the dye molecules will be released from their initial environment to the external solution. After the release the dyes must remain dissolved in the more hydrophilic media to fulfill their sensing function, see J. Zhou, D. Yao, Z. Qian, S. Hou, L. Li, A.T.A. Jenkins, Y. Fan: Bacteria-responsive intelligent wound dressing: simultaneous in situ detection and inhibition of bacterial infection for accelerated wound healing. Biomaterials 161, 11-23 (2018).

The compounds can have an additional coupling functionality for being covalently bound to an analyte or substrate, e.g. biological molecules like proteins, nanobodies, antibodies, enzymes, oligonucleotides, or polymers for the usage in wound dressings. The introduction of a great variety of different coupling groups can be easily facilitated by methods known to those skilled in the art via active ester chemistry (see Examples).

But even without a covalent coupling group the dyes can be applied in several ways, e.g. inside of vesicles, membranes etc. as a sensor or for the determination of the surrounding pH.

The use as a pH sensor in wound dressings can include the coupling of the pH-sensitive compounds to a carrier substance. Such carriers in dressing for chronic or complex wounds are often hydrogels based on chitin or chitosan.

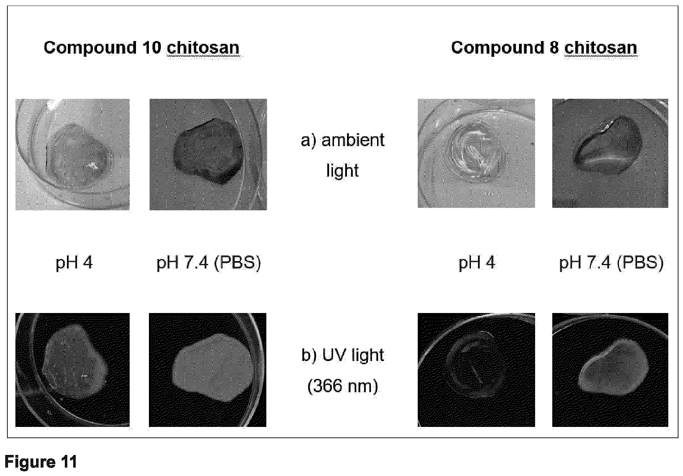

To demonstrate the suitability and functionality of the novel compounds as pH sensors under real world conditions compounds 8 and 10 were coupled via NHS-ester chemistry to chitosan. As can be seen in Figure 11, the color difference of both acidic and basic form of the chemically bound compounds in response to the pH of the surrounding media is clearly visible with the naked eye. The inventive compounds can therefore act as a chromogenic or colorimetric pH monitor via their pH dependent absorption.

In the case of compound 8 with a turn-on-ratio of about 29 the basic form has a much higher fluorescence in comparison with the acidic form. When irradiated with a standard UV-lamp ("black light torch"), this can be easily observed by the unaided eye as the chitosan film at pH 7.4 is much "brighter" compared with the film at pH 4. Compound 8 therefore can be used either as a colorimetric or as a fluorogenic pH sensor.

For the clinical or medical application, the visibility of the color change or the easy observation of a change in fluorescence intensity without sophisticated technical equipment is very beneficial. The real-time monitoring of pH changes in chronic wound, e.g. caused by pathogenic infection is a great advantage in comparison to the time consuming standard bacterial cultures taking place in laboratories away from the patient.

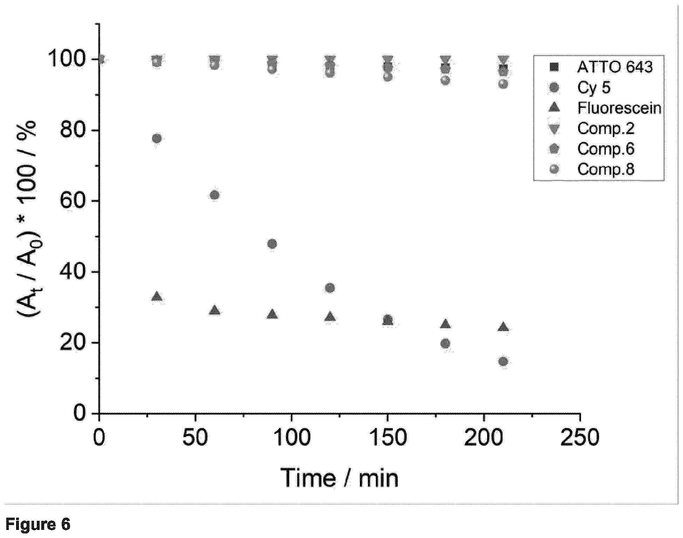

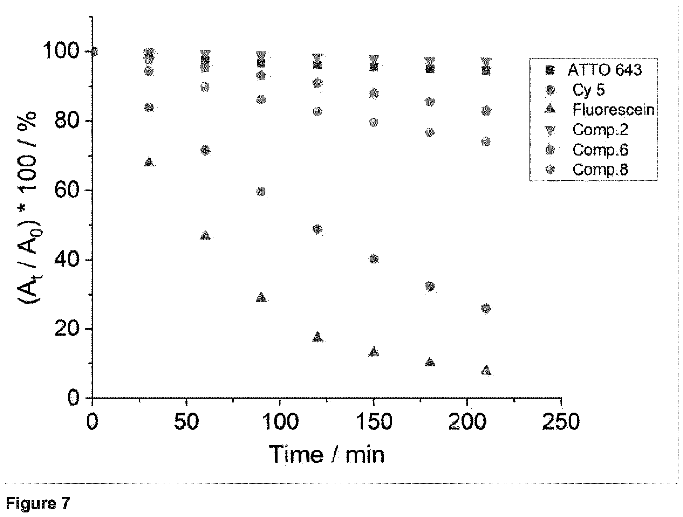

The photostability of the compounds is exceptionally high. It was measured under stress conditions by irradiation of both acidic and basic form in aqueous buffer solutions with a 575 W mercury halide lamp (see Figure 6 and Figure 7; for clarity, only inventive compounds 2, 6, and 8 are shown, which cover the range of photostability observed). The photostability of both forms were comparable to that of the very photostable, hydrophilic dye ATTO 643 and are much better than that of the also commercially available dyes Fluorescein and Cy 5. In particular, Fluorescein can act as direct comparison due to its common use as a pH-sensitive dye. The inventive compounds are much more photostable than Fluorescein.

This makes the compounds of the present invention ideal candidates not only for prolonged usage in wound dressings during the long-term monitoring of wound pH but also for the application in high resolution microscopy or nanoscopy, where high laser power for the excitation of fluorescent labels is used.

The compounds are derivatives of xanthene and therefore presumably have a similar low toxicity, see R. Alford, H.M. Simpson, J. Duberman, G.C. Hill, M. Ogawa, C. Regino, H. Kobayashi, P.L. Choyke: Toxicity of Organic Fluorophores Used in Molecular Imaging. Molecular Imaging 8(6), 341 (2009).

An important mercantile advantage of the present invention is the simple preparation of the compounds from commercially available or economically produced precursor compounds. This one-step synthesis facilitates the easy and versatile treatment of the precursor with a diluted hydroxide solution, e.g. for compound 22:

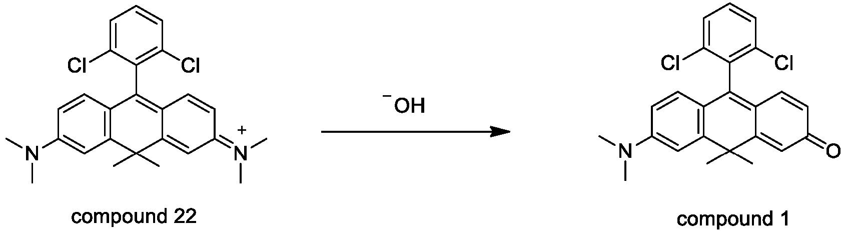

For that purpose, the direct precursor compound is dissolved in an aqueous hydroxide solution. Organic solvents as ethanol, methanol, acetone, or acetonitrile can be used as co-solvents, e.g. to improve the solubility of the reactants.

For the conversion any alkaline or alkaline earth hydroxide can be used, such as lithium, sodium, potassium, cesium, calcium or barium hydroxide. Lithium, sodium, and potassium hydroxide are preferred for their higher reactivity. Even mixtures of different hydroxides can be used.

The reaction time depends on the concentration of the reactants (precursor and alkali) and the temperature. A temperature of at least 0°C is necessary for the reaction to proceed in reasonable time, a faster and more uniform conversion is found in the temperature range of 25 - 100°C. The bests results were obtained with temperatures between 50 and 85°C.

The only prerequisite for that simple one-step reaction is the presence of a dimethyl amino group at at least one terminal end of the chromophoric system of the parent compound, as seen in the following examples of precursors applied for the preparation of compounds according to the invention:

**Table 3: Precursor compounds for the synthesis of compounds according to general formula (I).**

| Compound | Structure | Precursor of |
|---|---|---|
| 22 | 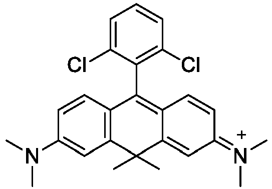 | compound 1 |
| 23 | 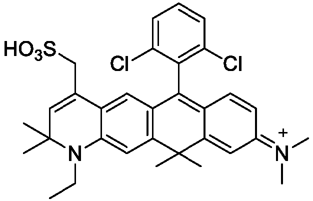 | compound 2 |
| 24 | 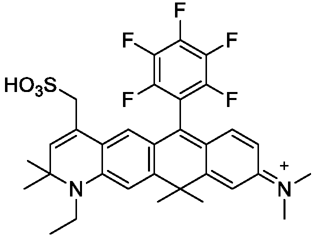 | compound 3 and compound 4; in an intermediate step the para-fluorine is substituted by taurine (compound 3) or 2-mercaptoethansulfonic acid (compound 4), see examples |
| 25 | 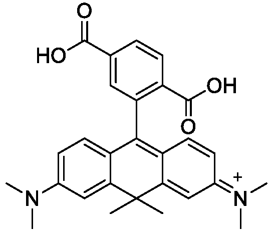 | compound 5 |
| 26 | 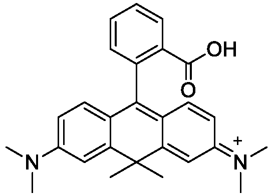 | compound 7 |
| 27 | 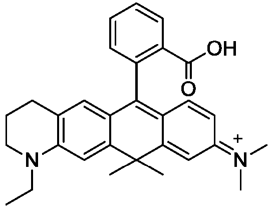 | compound 9 |

The precursor compounds can be prepared according to the literature and procedures known to the skilled in the art, e.g. J.L Bachmann, C.I. Pavlich, A.J. Boley, E.M. Marcotte, E. V Anslyn: Synthesis of Carboxy ATTO 647N Using Redox Cycling for Xanthone Access. Org. Lett. 22, 281 (2020).

A very important advantage of the present invention is therefore the almost simultaneous production of two valuable dyes, i.e. the precursor "carborhodamine" and the product "carborhodol", whereas with the literature method described in M. Sednev, C. Wurm, V. Belov, S. Hell, Carborhodol: A Hybrid Fluorophore Obtained by Combination of Fluorescein and Carbopyronin Dye Cores, Bioconjugate Chemistry 24, 690 (2013) only a single target compound - the carborhodol - is obtained in a multi-step synthesis.

Another subgroup of the compounds according to the present invention belong to the dye class of unsymmetrical carbazines, X = CR₁₁R₁₂ in general formulae (III) and (IV). These dyes can for example be prepared according to a synthesis route given in US4892858. Absolutely no reference is given within that patent about any other optical property than absorption. Nothing is mentioned about fluorescence or pH-dependent optical properties of these compounds, which were then used just as part of heat sensitive transfer materials.

The compounds show absorption and fluorescence at wavelengths above 560 nm in the red region of the visible spectrum (see Table 2). The many advantages of the red spectral region have already been described in detail above. The absorption and fluorescence wavelengths of the compounds can be easily tuned by the introduction of different substituents like additional ring systems. This can be seen by a comparison of compounds 12, 19, 20, and 21 in Table 2.

Furthermore, we were successful in introducing coupling groups. Compounds 15, 16, and 17 in Table 2 comprising a carboxylic acid group and can therefore be covalently bound to an analyte or substrate as discussed earlier.

With the introduction of hydrophilic or hydrophobic groups it is possible to change the solubility of the compounds according to the needs of the respective application.

Surprisingly these compounds exhibit a very large Stokes shift, i.e. the wavelength difference of absorption and fluorescence maximum, of usually about 50 - 120 nm (see Table 2). A large Stokes-shift is very beneficial for multiplexing applications, where various dyes are excited simultaneously at one single wavelength and their spectrally separated fluorescence is detected within different emission channels. The spectral separation of excitation light with optical filters is easier for dyes with a larger Stokes shift. This results in a better signal-to-noise ratio and therefore can lead to a higher detection sensitivity.

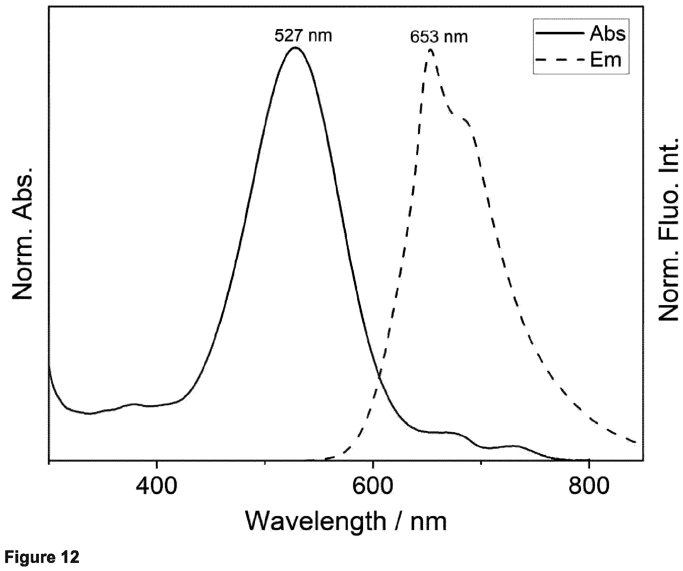

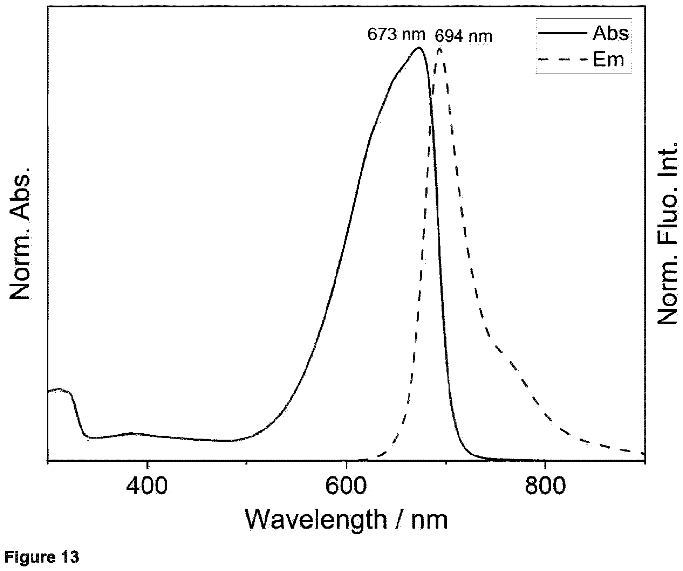

The absorption and fluorescence spectra of compound 11 are shown in Figure 12 (acidic form) and Figure 13 (basic form).

The measurement of the absorption spectrum at different pH clearly shows isosbestic points indicating the conversion between two defined species, i.e. the protonated (acidic) and deprotonated (basic) form, e.g. of compound 11:

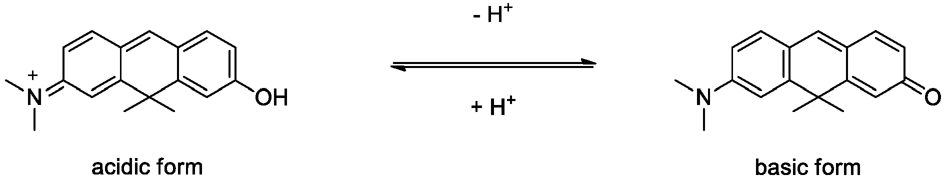

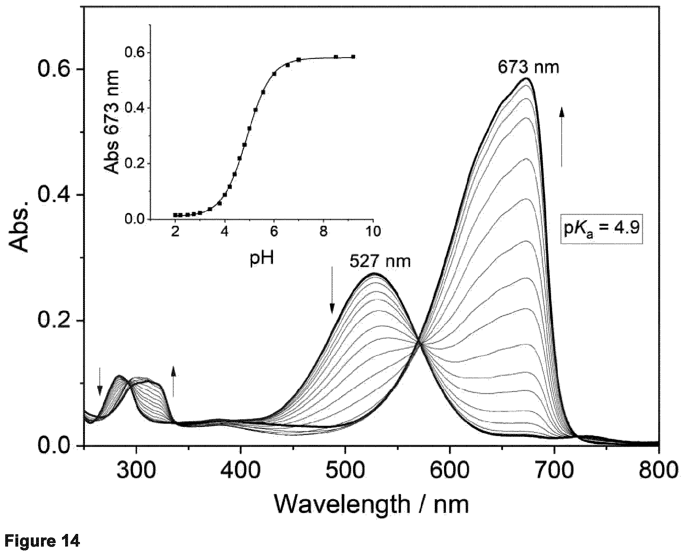

Due to the different absorption of both forms a color change according to the pH of the solution is visible with the naked eye. The compounds can therefore be easily used as pH sensors or indicator dyes for colorimetric measurements (see Figure 14).

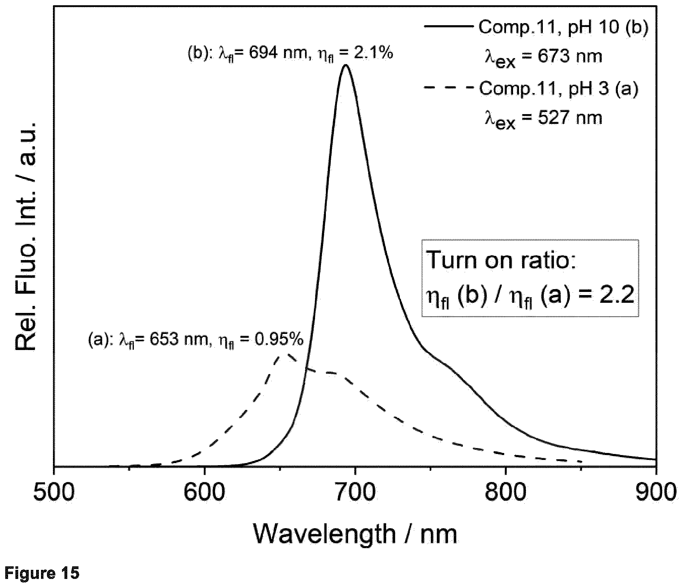

The fluorogenic behavior and turn-on-ratio of compound 11 is illustrated in Figure 15: The fluorescence quantum yield of the basic form is more than two times higher than that of the acidic form.

The compounds of the invention are produced in accordance with the procedures described in more detail in the following examples.

### Examples for the preparation of compounds according to the invention

### Compound 1 and 2 (containing a chloro-substituted head group)

The corresponding carborhodamine (compound 22 or 23) is dissolved in a 1:1 mixture of ethanol and 0.1 M aq. LiOH. The reaction mixture is stirred at 90°C overnight, during which the color changes from green to blue within the first hour. After the completion of the reaction, the organic layer is separated using dichloromethane acidified with trifluoroacetic acid. The organic layer is dried using sodium sulfate, filtered and the solvent is evaporated. The crude product is purified by chromatography yielding compound 1 (47%) or compound 2 (43%), respectively.

### Compound 3 and 4 (containing a fluoro-substituted head group)

Compound 24 is dissolved in DMSO and 5 equivalents of taurine sodium-salt or 2-mercaptoethansulfonic acid sodium-salt is added. The solution is stirred at 70°C for two hours. The intermediate compound is purified by column chromatography. The corresponding tetrafluorosubstituted carborhodamine is dissolved in a 0.05 M LiOH solution and subjected to stirring at 50°C for a duration of 96 hours. The color changes from green to blue after about 4 hours. After the completion of the reaction, the pH is adjusted to 5 by addition of trifluoroacetic acid. The resulting solution is subjected to column chromatography yielding compound 3 (59%) or compound 4 (46%), respectively.

### Compounds 5, 7 and 9 (containing a carboxlic acid head group)

The corresponding carborhodamine (compound 25, 26 or 27) is dissolved in a 1:1 mixture of ethanol and 2.5 M aq. NaOH. The resulting mixture is stirred at 85°C for 3 hours, during which the color of the solution changes from blue to red within 15 minutes.

Upon completion of the reaction, the mixture is cooled to 0°C and trifluoroacetic acid is added to achieve a pH of 4 to 5. After the addition the bright red color changes to a dull dark red. The product is extracted with dichloromethane and the organic layer dried with sodium sulfate, filtered and evaporated to dryness. The crude product is purified by chromatography yielding compound 5 (44%), compound 7 (68%) or compound 9 (54%), respectively.

### Compound 6

To a stirred solution of compound 5 (20 mg, 36 µmol) anhydrous acetonitrile containing p-toluenesulfonic acid (c = 5 g/L, 10 mL, stored over a molecular sieve 3 Å) at 0°C, N-hydroxysuccinimide (5.5 mg, 1.3 eq.) and N, N'-dicyclohexylcarbodiimide (DCC) (8.05 mg, 1.05 eq.) are added. A precipitate occurs immediately after the addition of DCC. The dull orange colored reaction mixture is stirred at 0°C overnight. The reaction mixture is filtered using a 0.2 µ membrane filter to remove the solids. After evaporating off the solvent a magenta-colored residue is obtained. The residue is redissolved in 2 mL of acetonitrile and the active ester of compound 5 is precipitated with diethyl ether and isolated as a red powder (19 mg, 98%).

The active-ester (20 mg, 38 µmol) is dissolved in 3 mL of dry DMSO and 4-(methylamino) butyric acid potassium salt (22 mg, 95 µmol) is added. The red-colored reaction mixture is stirred at room temperature for 2 hours. The reaction mixture is then added to 13 mL of distilled water while stirring at 0°C. The resulting solution is applied to a reverse phase column chromatography yielding compound 6 (98%).

### Compound 8

Compound 7 (100 mg, 0.2 mmol) is dissolved in 10 mL of dichloroethane, to which POCl₃ (951 µL, 10.2 mmol) is added. After stirring at 80°C for 4 hours, all volatile materials are evaporated in vacuo and the solid residue is dissolved in 5 mL acetonitrile. A solution of piperidine-4-carboxylic acid (129 mg, 1 mmol) in 5 mL acetonitrile is added, followed by the addition of triethylamine (842 µL, 6 mmol). After stirring at room temperature for 30 min, all volatile materials are removed in vacuo. The residue is redissolved in 20 ml dichloromethane and washed with a saturated aqueous solution of ammonium chloride. The organic layer is separated, dried with sodium sulfate, and the organic solvent is evaporated off. Column chromatography yielded compound 8 (60%).

### Compound 10

This compound is synthesised starting from compound 9 according to the procedure given for compound 8 above. Methyl-4-(methylamino)butanoate is used instead of piperidine-4-carboxylic acid.

For the hydrolysis of the ester group, the crude product is stirred for 5 h at room temperature in a mixture of H₂O:THF:1 M aq. NaOH (22 mL:34 mL:1.4mL). Acetic acid (23 mL) is added, and the mixture is evaporated to dryness. Purification of the crude product by column chromatography yielded compound 10 (67%).

### Compound 17

3-(Hydroxydiphenylmethyl)phenol is prepared from ethyl 3-hydroxybenzoate and phenyl magnesium bromide in diethyl ether in a standard Grignard reaction. N-methyl-4-nitroanline and ethyl 4-bromobutanoate are refluxed together with N-ethyl-N-isopropylpropan-2-amine in acetonitrile to yield ethyl 4-(methyl(4-nitrophenyl)amino)butanoate. The yellow product is isolated as a solid by column chromatography on silica with a pentane/chloroform/methanol gradient. The conversion of the nitro group to the amino group is carried out in the presence of powdered iron and a small amount of hydrochloric acid in boiling water. The resulting phenylenediamine compound is purified by column chromatography. Equimolar amounts of 3-(Hydroxydiphenylmethyl)phenol and ethyl 4-((4-aminophenyl)(methyl)amino)butanoate are mixed together with sodium carbonate in a 1:5 mixture of ethanol and water at room temperature. An aqueous solution of ammonium persulfate is added to facilitate the intermediate oxidation of the phenylenediamine. After half an hour the dark blue solution is extracted with ethyl acetate. The blue extract is treated with aqueous sodium dithionite to get the leuco form of the indoaniline intermediate. The organic phase is dried with sodium sulfate, filtered and evaporated to dryness.

The oily residue is redissolved in chloroform and the solution is cooled to 0°C. Sulfuric acid is added dropwise followed by the removal of chloroform in vacuo. The remaining mixture is stirred at room temperature for 1 hour to complete the ring closure. A cooled aqueous sodium hydroxide solution is added to adjust the pH to 5 - 6. Then chloroform and an aqueous sodium periodate solution are added to the mixture. The phases are separated, and the aqueous layer is extracted twice with chloroform. The chloroform extracts are pooled, dried with sodium sulfate, filtered and evaporated to dryness. The intermediate is purified by column chromatography.

For the hydrolysis of the ester group, the intermediate is refluxed with a fourfold molar quantity of hydrochloric acid for 5 h in a 1:1 mixture of water and acetone. Purification of the crude product by column chromatography yielding compound 17 (20 %).

### Compound 18

2-(3-Dimethylamino)phenyl)propan-2-ol is prepared from ethyl 3-(dimethyamino)benzoate and methyl magnesium iodide in diethyl ether in a standard Grignard reaction.

Equimolar amounts of 2-(3-Dimethylamino)phenyl)propan-2-ol and 2,6-Dichloro-4-(chloroimino)cyclohexa-2,5-dienone (Gibb's reagent) are mixed together with sodium carbonate in a 1:10 mixture of ethanol and water at room temperature. After half an hour the greenish black solution is extracted with ethyl acetate. The blue extract is treated with aqueous sodium dithionite to get the leuco form of the indoaniline intermediate. The organic phase is dried with sodium sulfate, filtered and evaporated to dryness.

The oily residue is redissolved in chloroform and the solution is cooled to 0°C. Sulfuric acid is added dropwise followed by the removal of chloroform in vacuo. The remaining bluish black mixture is stirred at room temperature for 2 hours to complete the ring closure. Ice and aqueous sodium hydroxide solution is added to adjust the pH to 5 - 6. Then chloroform and an aqueous sodium periodate solution are added to the mixture. The phases are separated, and the aqueous layer is again extracted with chloroform. The chloroform extracts containing the target compound are pooled, dried with sodium sulfate, filtered and evaporated to dryness. The black residue can be purified by column chromatography yielding compound 18 (25%).

### Brief description of the drawings

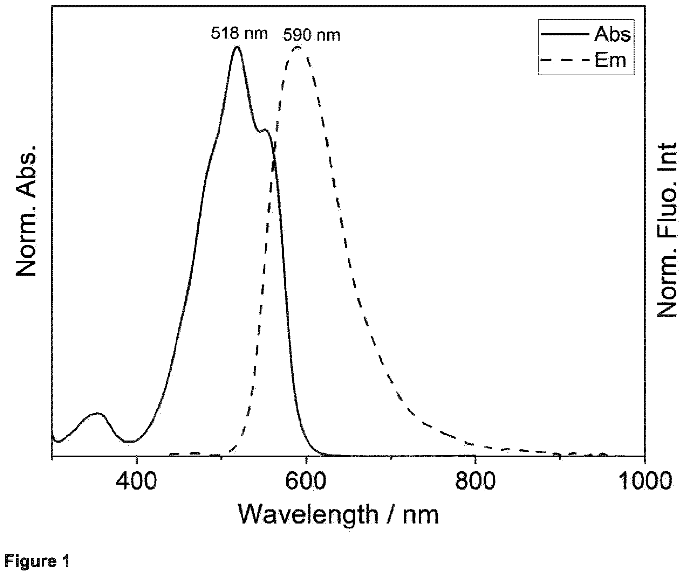

**Figure 1****:** Compound 1, acidic form: Absorption and emission spectrum in buffer pH 3 at 25°C.

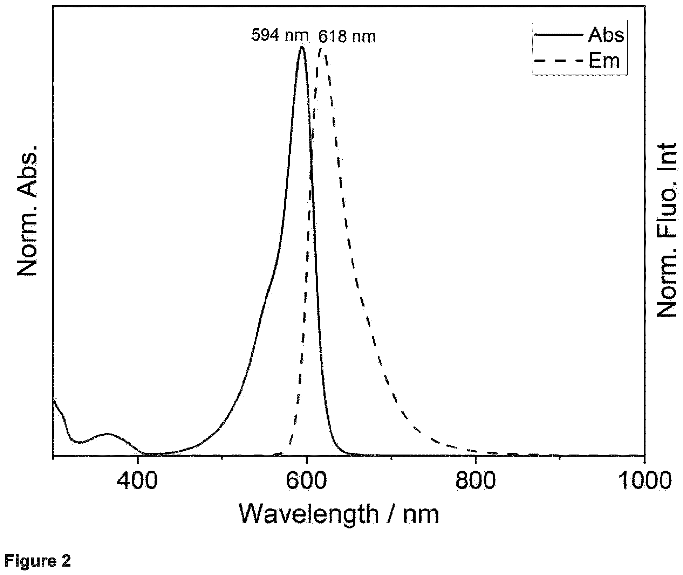

**Figure 2****:** Compound 1, basic form: Absorption and emission spectrum in buffer pH 1 0 at 25°C.
**Figure 3****:** Compound 1: Spectroscopic determination of p*K*ₐ in aqueous solution at 25°C by measuring the absorption spectrum at different pH.
**Figure 4****:** Compound 1, acidic and basic form: Fluorescence spectra, fluorescence quantum yield and turn-on-ratio, i.e. the ratio of the fluorescence quantum yields of basic and acidic form (buffer pH 10 and buffer pH 3 at 25°C).
**Figure 5****:** Compound 3: Relative fluorescence intensity in aqueous buffer solutions of different pH at 25°C.
**Figure 6****:** Compounds 1, 6 and 8, acidic form: Photostability in comparison to commercial dyes (ATTO 643, Fluorescein, Cy 5) in buffer pH 3 at 25°C.
**Figure 7****:** Compounds 1, 6 and 8, basic form: Photostability in comparison to commercial dyes (ATTO 643, Fluorescein, Cy 5) in buffer pH 10 at 25°C.
**Figure 8****:** Compound 1, basic form: Solvatochromism. Normalized absorption spectra in different solvents in accordance with the E_{T}(30)-scale of solvent polarity at 25°C.
**Figure 9****:** Compound 1, basic form: Solvatochromism. Normalized fluorescence spectra in different solvents in accordance with the E_{T}(30)-scale of solvent polarity at 25°C.
**Figure 10****:** Hydrophobicity of compounds 1, 2 and 3 in comparison to commercial dyes (ATTO 643, ATTO 647N, Fluorescein).
**Figure 11****:** Chitosan films of compound 10 and compound 8 as a model for wound dressing wetted with aqueous solutions of different pH. Further explanation see text.
   a) Under ambient light the pH-dependent color change of both compounds is clearly visible.
   b) Under irradiation with UV light (366 nm) the fluorogenic behavior of compound 8 with a turn-on-ratio of about 29 is clearly visible.
**Figure 12****:** Compound 11, acidic form: Absorption and emission spectrum in buffer pH 3 at 25°C.
**Figure 13****:** Compound 11, basic form: Absorption and emission spectrum in buffer pH 10 at 25°C.
**Figure 14****:** Compound 11: Spectroscopic determination of p*K*ₐ in aqueous solution at 25°C by measuring the absorption spectrum at different pH.
**Figure 15****:** Compound 11, acidic and basic form: Fluorescence spectra, fluorescence quantum yield and turn-on-ratio, i.e. the ratio of the fluorescence quantum yields of basic and acidic form (buffer pH 10 and buffer pH 3 at 25°C).

The present application also provides the following items:
Item 1. A dye having pH-dependent absorption and/or pH-dependent fluorescence having general formula (I), (II), (III) or (IV) 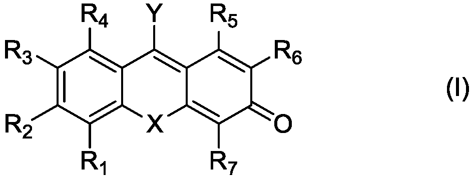 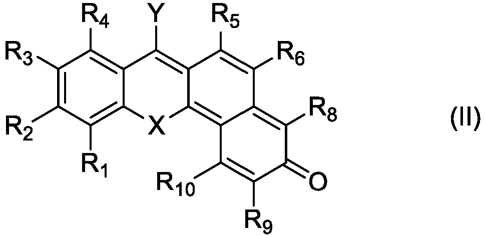 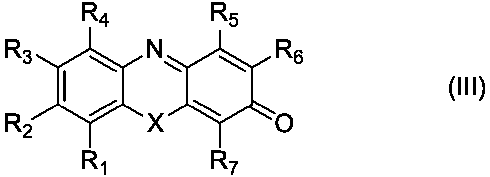 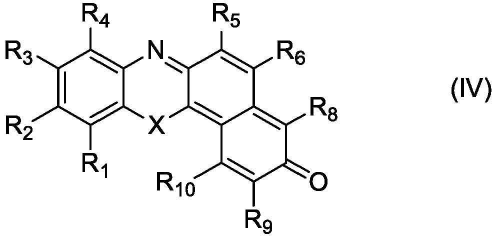 wherein
   X = CR₁₁R₁₂, SiR₁₃R₁₄, sulfur, SO₂ or P(O)OR₁₅,
   Y = hydrogen, a nitril group, a carboxyl group, a carboxylic acid derivative, an amino group or a straight-chain, branched or cyclic, saturated or unsaturated hydrocarbon moiety having up to 40 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
   R₁, R₃, R₄, R₅, R₆, R₇, R₈, R₉, R₁₀ independently are hydrogen, halogen, a hydroxyl group, thiol group, amino group, sulfo group, phospho group, nitro group, carbonyl group, carboxyl group, a carboxylic acid derivative, a nitrile group, isonitrile group, cyanate group, thiocyanate group, isothiocyanate group or a straight-chain, branched or cyclic saturated or unsaturated hydrocarbon moiety having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
   R₁₁, R₁₂, R₁₃, R₁₄, R₁₅, R₃₁, R₃₂ independently are hydrogen or a hydrocarbon group having 1-20 carbon atoms, wherein the hydrocarbon group optionally comprises one or more heteroatoms selected from the group of N, O, and S, or/and one or more substituents preferably selected from the group consisting of halogen, nitrile group, hydroxyl group, thiol group, amino group, nitro group, sulfo group, phospho group, carbonyl group, carboxyl group, a carboxylic acid derivative,
   or R₁₁ and R₁₂ or R₁₃ and R₁₄, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, CONR₃₁R₃₂ and a hydrocarbon group having from 1 - 20 carbon atoms or/and can be fused with one or more 3- to 7-membered rings, wherein the hydrocarbon group optionally comprises one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SO₃H, or CONR₃₁R₃₂;
   R₂ = NR₁₆R₁₇ or R₁ together with R₂ is 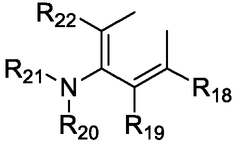
   in which R₁₈, R₁₉ and R₂₂ are defined as R₁ and
   R₁₆, R₁₇, R₂₀, R₂₁ are defined as R₁₁
   or wherein at least one of R₁-R₂₂ forms a ring system with one or more neighboring moieties.
Item 2. A compound having general formula (I), (II), (III) or (IV) 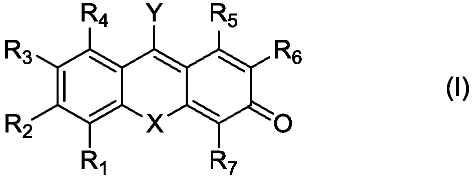 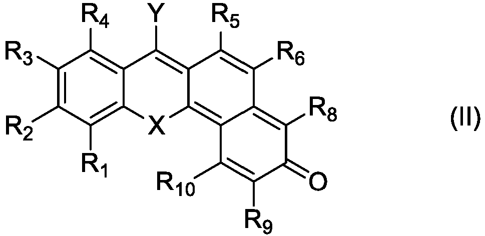 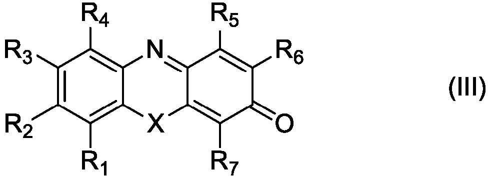 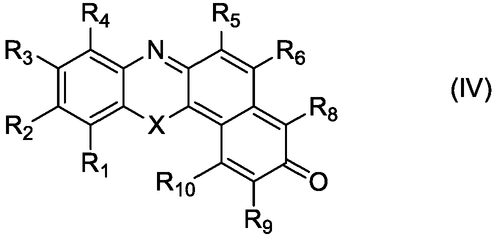 wherein
   X = CR₁₁R₁₂, SiR₁₃R₁₄, sulfur, SO₂ or P(O)OR₁₅,
   Y = hydrogen, a nitril group, a carboxyl group, a carboxylic acid derivative, an amino group or a straight-chain, branched or cyclic, saturated or unsaturated hydrocarbon moiety having up to 40 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
   R₁, R₃, R₄, R₅, R₆, R₇, R₈, R₉, R₁₀ independently are hydrogen, halogen, a hydroxyl group, thiol group, amino group, sulfo group, phospho group, nitro group, carbonyl group, carboxyl group, a carboxylic acid derivative, a nitrile group, isonitrile group, cyanate group, thiocyanate group, isothiocyanate group or a straight-chain, branched or cyclic saturated or unsaturated hydrocarbon moiety having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂ and SOsH, or CONR₃₁R₃₂;
   R₁₁, R₁₂, R₁₃, R₁₄, R₁₅, R₃₁, R₃₂ independently are hydrogen or a hydrocarbon group having 1-20 carbon atoms, wherein the hydrocarbon group optionally comprises one or more heteroatoms selected from the group of N, O, and S, or/and one or more substituents preferably selected from the group consisting of halogen, nitrile group, hydroxyl group, thiol group, amino group, nitro group, sulfo group, phospho group, carbonyl group, carboxyl group, a carboxylic acid derivative,
   or R₁₁ and R₁₂ or R₁₃ and R₁₄, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SO₃H, CONR₃₁R₃₂ and a hydrocarbon group having from 1 - 20 carbon atoms or/and can be fused with one or more 3- to 7-membered rings, wherein the hydrocarbon group optionally comprises one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
   R₂ = NR₁₆R₁₇ or R₁ together with R₂ is 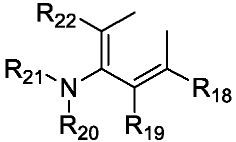
   in which R₁₈, R₁₉ and R₂₂ are defined as R₁ and
   R₁₆, R₁₇, R₂₀, R₂₁ are defined as R₁₁
   or wherein at least one of R₁-R₂₂ forms a ring system with one or more neighboring moieties,
   with the proviso,
   that in a compound of formula (I) there are not simultaneous:
      X = C(CH₃)₂;
      R₂ = N(CH₃)₂; and
      Y = 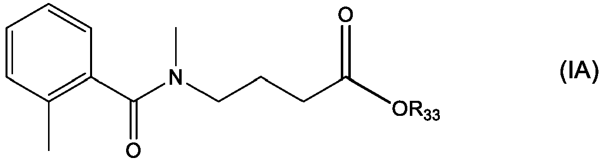
      wherein R₃₃ = H or CH_{3;} and
      that in a compound of formula (I) there are not simultaneous
      X = C(CH₃)₂;
      R₂ = N(CH₃)₂; and
      Y = 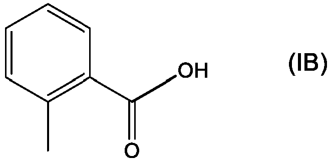 and
      that in a compound of formula (I) there are not simultaneous
      X = C(CH₃)₂;
      R₂ = N(CH₃)₂; and
      Y = 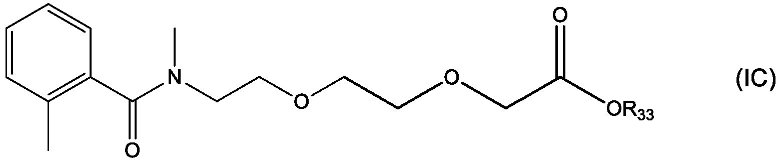
      wherein R₃₃ = H or CH_{3;} and
      that in a compound of formula (I) there are not simultaneous
      X = Si(CH₃)₂;
      R₂ = NH₂, NH(CH₃), N(CH₃)₂ or NH-C(O)CH₃; and
      Y = 1-methyl-phenyl,
      and wherein compounds of formula (III) with X = CR₁₁R₁₂ comprise at least one sulfo group and in particular wherein in compounds of formula (III) with X = CR₁₁ R₁₂ at least one of R₁, R₃, R₄, R₅, R₆ or R₇ = SO₃H.
Item 3. The dye or compound of item 1 or 2, wherein R₁ and R₁₆ and/or R₃ and R₁₇ form a ring system resulting in one of the following structures (A), (B), (C), (D) (E), (F) and (G) 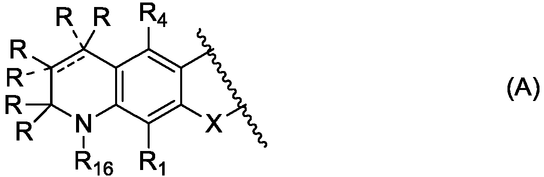 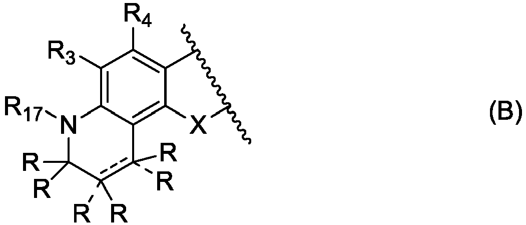 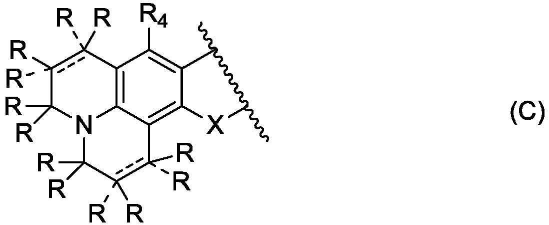 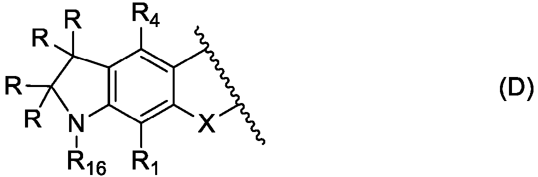 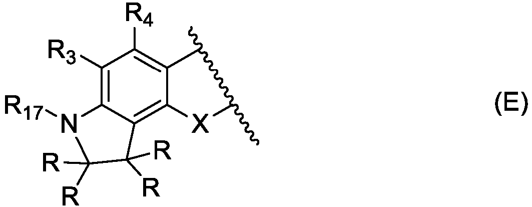 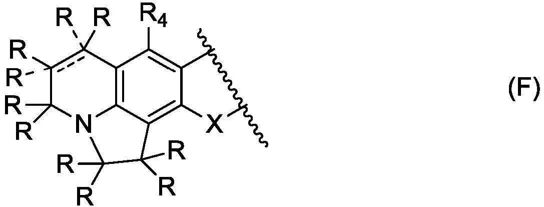 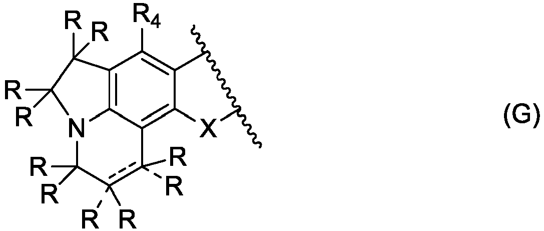
   in which R in each case independently is defined as R₁ and the dashed lines are optionally double bonds in the presence of which the moieties bound via a dashed line are absent; and whereby
   neighboring substituents R may optionally form further ring systems which comprise 5- and/or 6-membered rings which may optionally contain further heteroatoms or substituents.
Item 4. The dye or compound of any one of items 1-3, wherein R3 and R17 and/or R1 and R16 form a ring system 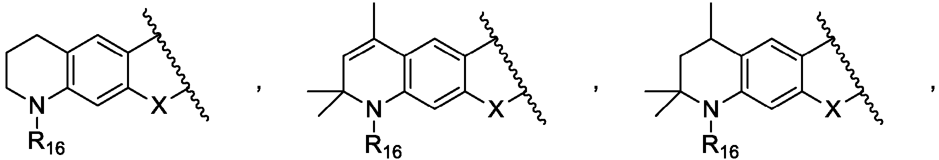 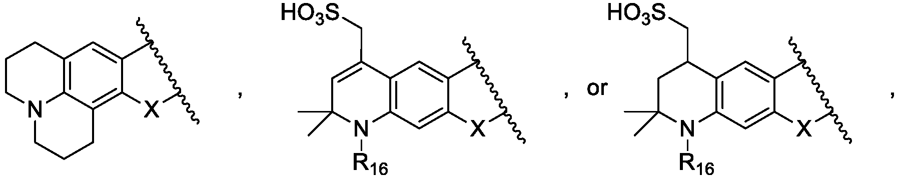 wherein R at each occurrence independently is defined as R₁
Item 5. The dye or compound of any one of items 1-4, wherein R₃ and R₄ and/or R₅ and R₆ and/or R₆ and R₈ form an annulated ring system selected from: 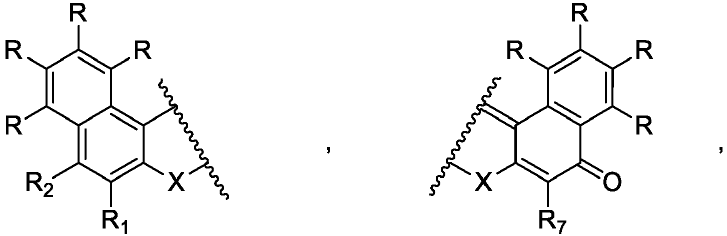 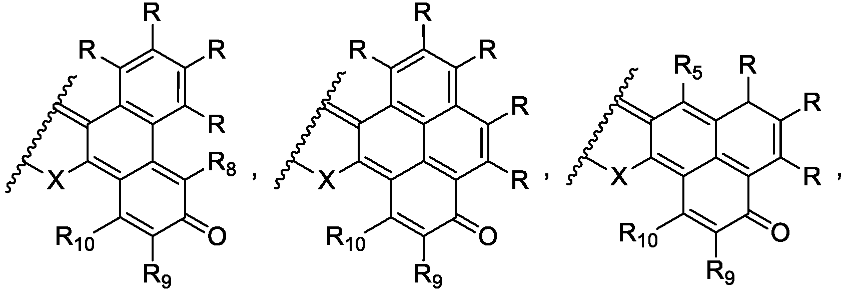 wherein R at each occurrence independently is defined as R₁.
Item 6. The dye or compound of any one of items 1-5, wherein Y in formulae (I) and (II) is optionally substituted cycloalkyl, optionally substituted phenyl, optionally substituted naphthyl or optionally substituted heteroaryl having one of the following structures (H), (J), (K), (L), (M), (N), and (O) 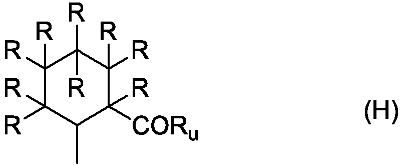 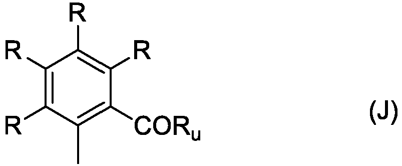 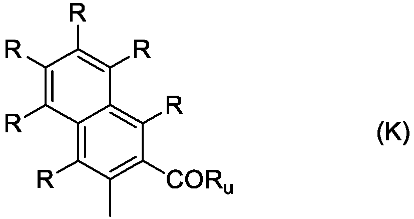 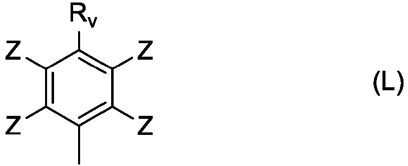 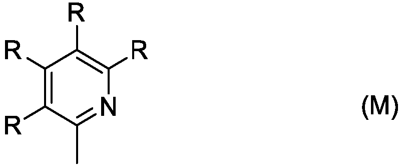 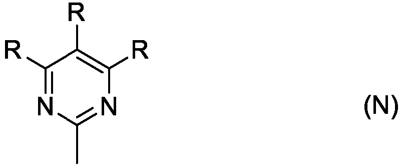 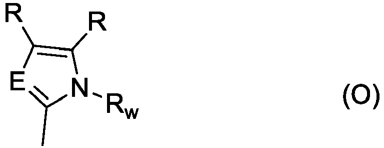 wherein
   E = oxygen, sulfur, nitrogen or ⁺NR₂₃
   Rᵤ = halogen, OR₂₄, NHR₂₅, NR₂₆R₂₇,
   Z = hydrogen or halogen,
   Rᵥ = Z, S(CH₂)ₓCOOH, S(CH₂)ₓSO₃H, NHR₂₈, NR₂₉R₃₀ and
   R_{w} =hydrogen, alkyl, (CH₂)ₓCOOH, (CH2)ₓSO₃H, (CH₂)ₓOH, (CH₂)ₓSH, (CH2)ₓNH2, (CH2)ₓZ with x = 1, 2, 3, 4, 5, 6,
   R₂₃, R₂₄, R₂₅, R₂₆, R₂₇, R₂₈, R₂₉, R₃₀ are independently of one another a straight-chain, branched or cyclic, saturated or unsaturated hydrocarbon moiety (such as alkyl, cycloalkyl, aryl, heteroaryl) having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
   and R at each occurrence independently is defined as R₁.
Item 7. The dye or compound of any one of items 1-6, wherein Y is selected from 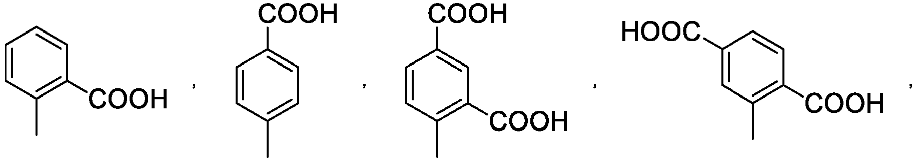 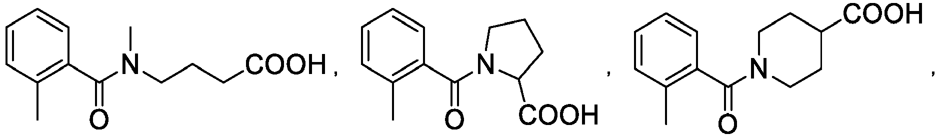 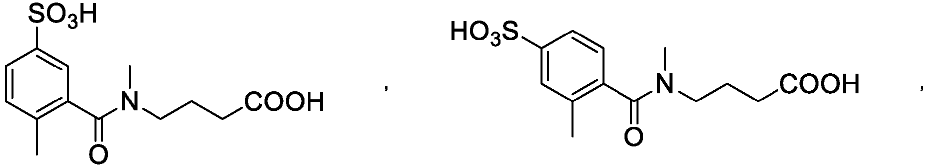 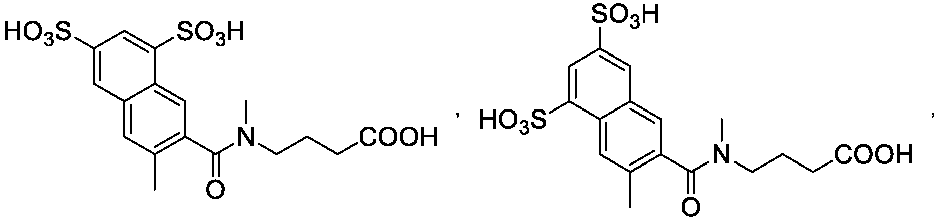 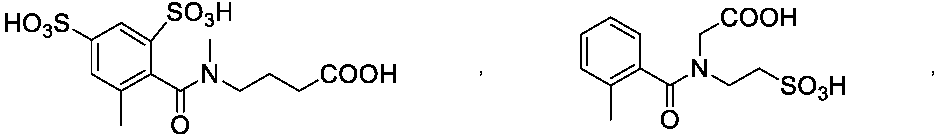 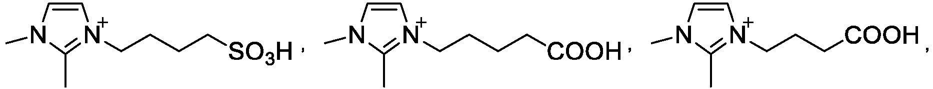 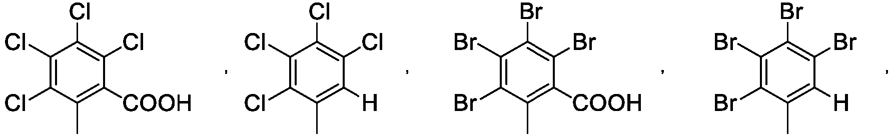 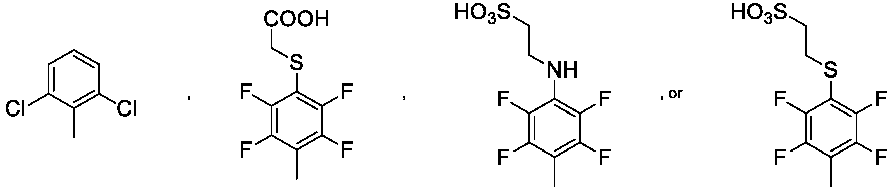
Item 8. The dye or compound of any of items 1-7, wherein R₁, R₃, R₄, R₅, R₆ and R₇ = H.
Item 9. The dye or compound of any one of items 1-7, wherein at least one of R₁, R₃, R₄, R₅, R₆ and R₇ = a substituent containing a COOH-group, a SOsH-group, and/or a halogen, in particular Cl or F.
Item 10. The dye or compound of any one of items 1-7, wherein R₃ is a substituent containing a COOH-group, a SOsH-group, in particular CH₂-SOsH, and/or a halogen, in particular Cl or F.
Item 11. The dye or compound of any one of items 1-10, wherein R₂ is N(CH₃)₂.
Item 12. The dye or compound of any one of items 1-10, wherein R₂ = NR₁₆R₁₇, wherein R₁₇ = C1-C4 alkyl, most preferably R₁₇ = CH₂-CH₃ and R₃ and R₁₆ together form a ring system having formula: 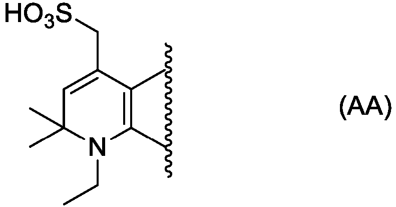
Item 13. The dye or compound of any one of items 1-10, wherein R₂ = NR₁₆R₁₇, wherein R₁₇ = C1-C4 alkyl, preferably R₁₇ = CH₂-CH₃ and R₃ and R₁₆ together form a ring system having the formula: 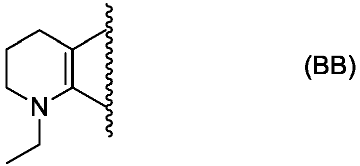
Item 14. The dye or compound of any one of items 1-13 comprising at least one SOsH group.
Item 15. The dye or compound according of any one of items 1-14 comprising at least one COOH group.
Item 16. The dye or compound of any one of items 1-15, wherein Y comprises a phenyl, naphthyl or imidazole group.
Item 17. They dye or compound of any one of items 1-15, wherein X = SIR₁₃R₁₄ and wherein Y is not 1-methyl-phenyl.
Item 18. Use of a dye or compound of any one of items 1-17 as pH sensor.
Item 19. pH-sensor comprising a dye or compound of any one of items 1-17.
Item 20. pH-sensor according to item 19 where the dye or compound is either used in solution; or incorporated in vesicles, polymerosomes or similar self-assembled compartments; or covalently bound or attached to the analyte or a substrate or support.
Item 21. Wound dressing comprising a dye or compound of any one of items 1-17.
Item 22. Use of a dye or compound of any one of items 1 - 17 as a marker group for the qualitative and/or quantitative determination of an analyte in a sample.
Item 23. Use according to item 22 where the analyte is a biomolecule, in particular an antibody, a nanobody, a peptide, a polypeptide, a protein, a nucleotide, a polynucleotide, a nucleoside, a nucleic acid, a nucleic acid analogue or a hapten.
Item 24. Use according to item 22 where the dye is covalently attached to the analyte.
Item 25. A method of preparing a dye or compound having formula (I), (II), (III) or (IV) as defined in any one of items 1-17 comprising the conversion of a precursor compound by a one-step one-pot synthesis using an aqueous alkaline or alkaline earth hydroxide solution as reaction medium.
Item 26. The method of item 25, wherein the alkaline or alkaline earth hydroxide is selected from lithium, sodium, potassium, cesium, calcium, or barium hydroxide or mixtures of different hydroxides.
Item 27. The method of item 25 or 26, wherein the conversion is performed at a temperature of at least 0°C, more preferably at a temperature range of from 25 to 100°C, or from 50 to 85°C.
Item 28. The method of any one of items 25-27, wherein the conversion is performed in presence of a co-solvent, preferably selected from the group of acetone, acetonitrile, ethanol, or methanol.
Item 29. The method of any one of items 25-28, wherein the precursor is a carborhodamine.
Item 30. The method of any one of items 25-29 comprising the step: 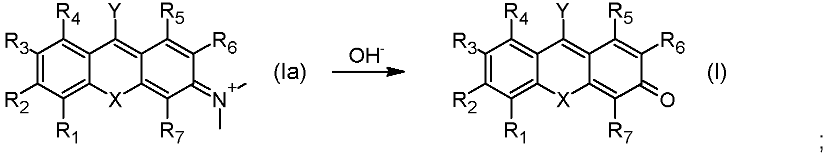 or 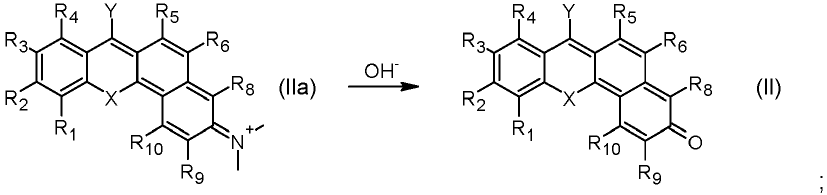 or 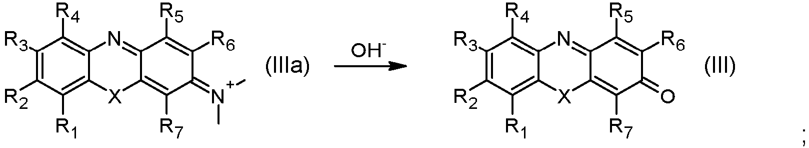 or 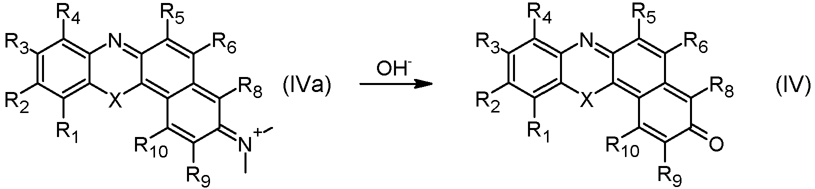
Item 31. Wound dressing according to item 21, wherein the dye or compound of any one of items 1-17 is incorporated in self-assembled liposomes, polymersomes, vesicles or capsules.
Item 32. Wound dressing according to item 21 or 31, wherein the dye or compound of any one of items 1-17 is coupled, in particular covalently coupled to a carrier or support.
Item 33. Wound dressing according to any one of items 21 or 31-32, wherein the wound dressing comprises a supporting material, in particular when the supporting material comprises chitin and/or chitosan.
Item 34. Wound dressing according to any one of items 21 and 31-33, wherein the supporting material comprises nanofibers, nanosheet bandage, composite films, nanoparticle clusters, microneedles, sponges and/or foams.
Item 35. Wound dressing according to any one of items 21 and 31-34, wherein the wound dressing comprises a carrier including a hydrogel based on chitin or chitosan.

## Claims

1. A dye having pH-dependent absorption and/or pH-dependent fluorescence having general formula (I), (II), (III) or (IV) 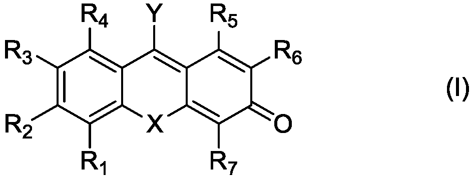 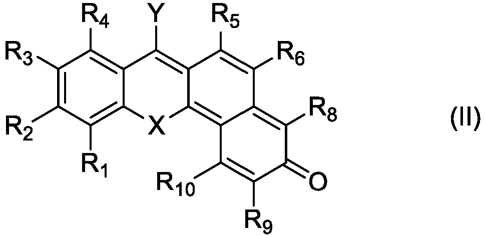 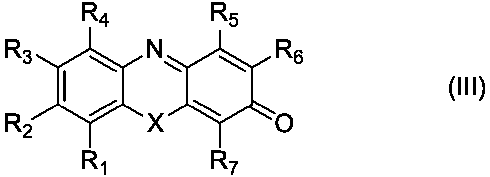 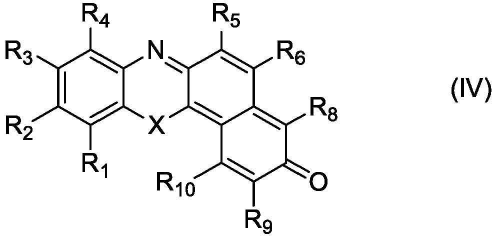 wherein
X = CR₁₁R₁₂, SiR₁₃R₁₄, sulfur, SO₂ or P(O)OR₁₅,
Y = hydrogen, a nitril group, a carboxyl group, a carboxylic acid derivative, an amino group or a straight-chain, branched or cyclic, saturated or unsaturated hydrocarbon moiety having up to 40 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
R₁, R₃, R₄, R₅, R₆, R₇, R₈, R₉, R₁₀ independently are hydrogen, halogen, a hydroxyl group, thiol group, amino group, sulfo group, phospho group, nitro group, carbonyl group, carboxyl group, a carboxylic acid derivative, a nitrile group, isonitrile group, cyanate group, thiocyanate group, isothiocyanate group or a straight-chain, branched or cyclic saturated or unsaturated hydrocarbon moiety having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
R₁₁, R₁₂, R₁₃, R₁₄, R₁₅, R₃₁, R₃₂ independently are hydrogen or a hydrocarbon group having 1-20 carbon atoms, wherein the hydrocarbon group optionally comprises one or more heteroatoms selected from the group of N, O, and S, or/and one or more substituents preferably selected from the group consisting of halogen, nitrile group, hydroxyl group, thiol group, amino group, nitro group, sulfo group, phospho group, carbonyl group, carboxyl group, a carboxylic acid derivative,
or R₁₁ and R₁₂ or R₁₃ and R₁₄, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, CONR₃₁R₃₂ and a hydrocarbon group having from 1 - 20 carbon atoms or/and can be fused with one or more 3- to 7-membered rings, wherein the hydrocarbon group optionally comprises one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SO₃H, or CONR₃₁R₃₂;
R₂ = NR₁₆R₁₇ or R₁ together with R₂ is 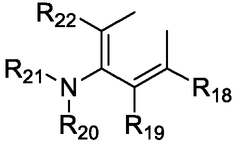
in which R₁₈, R₁₉ and R₂₂ are defined as R₁ and
R₁₆, R₁₇, R₂₀, R₂₁ are defined as R₁₁
or wherein at least one of R₁-R₂₂ forms a ring system with one or more neighboring moieties.

2. The dye of claim 1, wherein R₁ and R₁₆ and/or R₃ and R₁₇ form a ring system resulting in one of the following structures (A), (B), (C), (D), (E), (F) and (G) 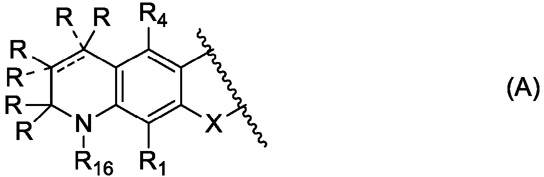 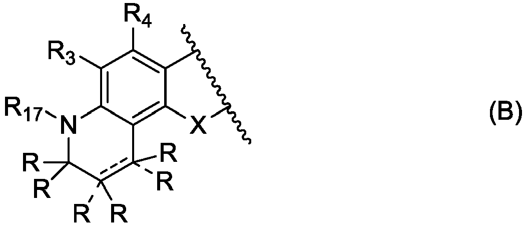 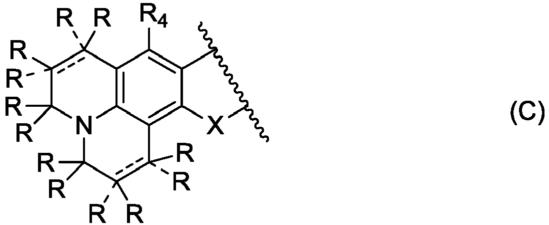 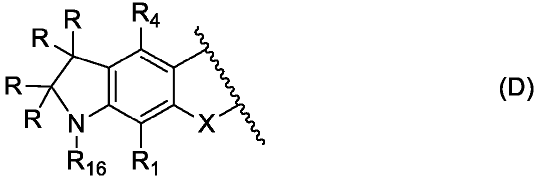 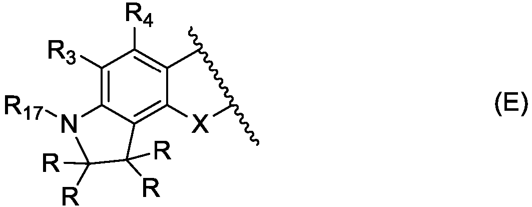 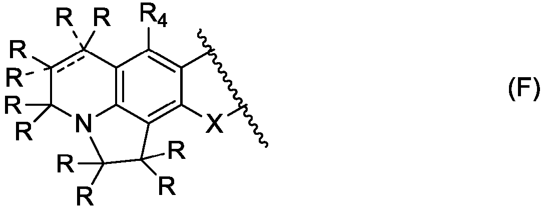 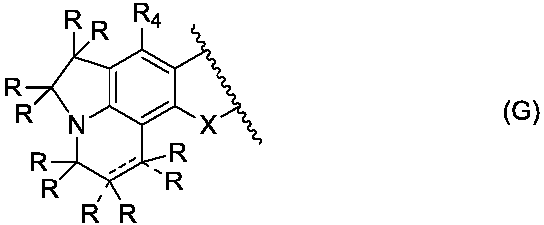
in which R in each case independently is defined as R₁ and the dashed lines are optionally double bonds in the presence of which the moieties bound via a dashed line are absent; and whereby
neighboring substituents R may optionally form further ring systems which comprise 5- and/or 6-membered rings which may optionally contain further heteroatoms or substituents.

3. The dye of claim 1 or 2, wherein R₃ and R₁₇ and/or R₁ and R₁₆ form a ring system 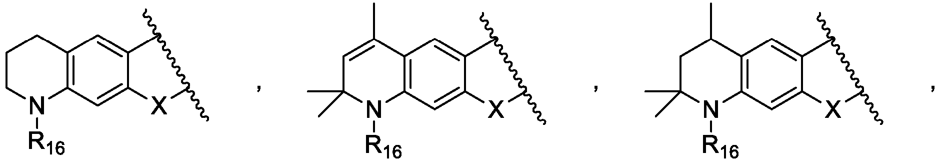 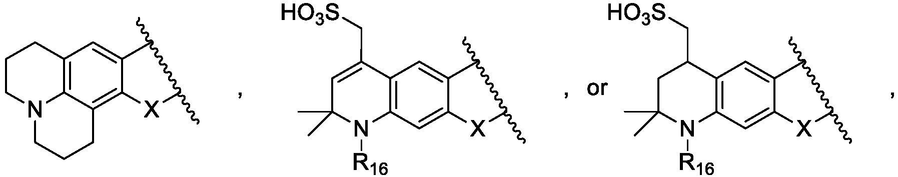 wherein R at each occurrence independently is defined as R₁

4. The dye of any one of claims 1-3, wherein R₃ and R₄ and/or R₅ and R₆ and/or R₆ and R₈ form an annulated ring system selected from: 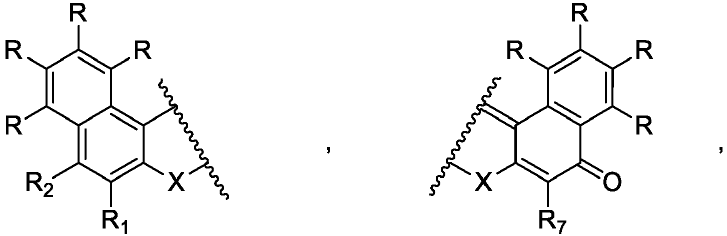 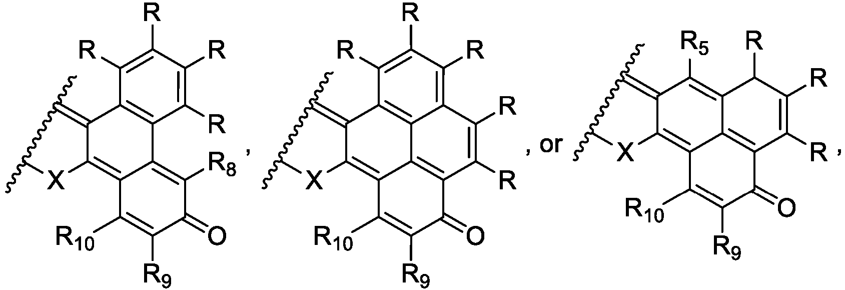 wherein R at each occurrence independently is defined as R₁.

5. The dye of any one of claims 1-4, wherein Y in formulae (I) and (II) is optionally substituted cycloalkyl, optionally substituted phenyl, optionally substituted naphthyl or optionally substituted heteroaryl having one of the following structures (H), (J), (K), (L), (M), (N), and (O) 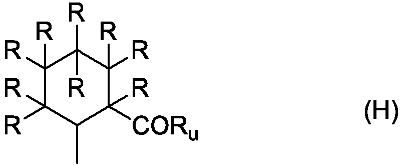 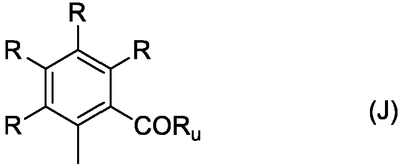 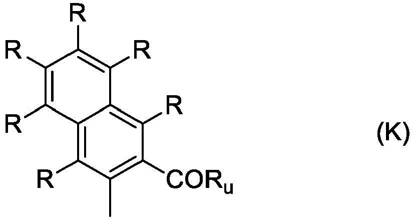 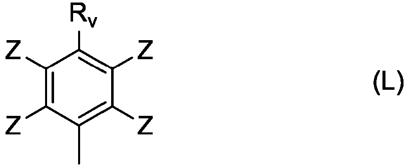 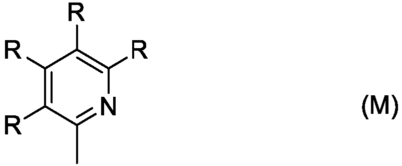 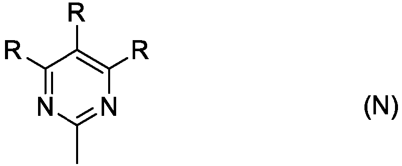 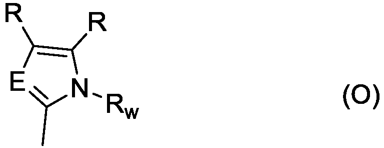 wherein
E = oxygen, sulfur, nitrogen or ⁺NR₂₃
Rᵤ = halogen, OR₂₄, NHR₂₅, NR₂₆R₂₇,
Z = hydrogen or halogen,
Rᵥ = Z, S(CH₂)ₓCOOH, S(CH₂)ₓSO₃H, NHR₂₈, NR₂₉R₃₀ and
R_{w} =hydrogen, alkyl, (CH₂)ₓCOOH, (CH2)ₓSO₃H, (CH₂)ₓOH, (CH₂)ₓSH, (CH2)ₓNH2, (CH2)ₓZ with x = 1, 2, 3, 4, 5, 6,
R₂₃, R₂₄, R₂₅, R₂₆, R₂₇, R₂₈, R₂₉, R₃₀ are independently of one another a straight-chain, branched or cyclic, saturated or unsaturated hydrocarbon moiety (such as alkyl, cycloalkyl, heterocycloalkyl, aryl, heteroaryl) having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SO₃H or CONR₃₁R₃₂;
and R at each occurrence independently is defined as R₁.

6. The dye of any one of claims 1-5, wherein Y is selected from

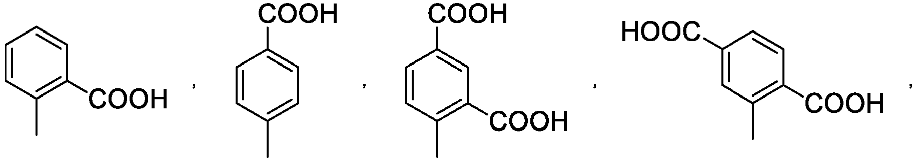

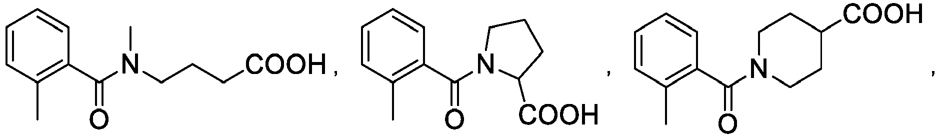

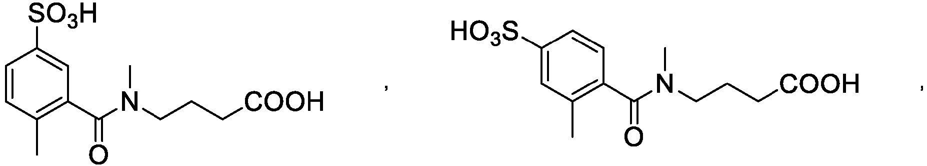

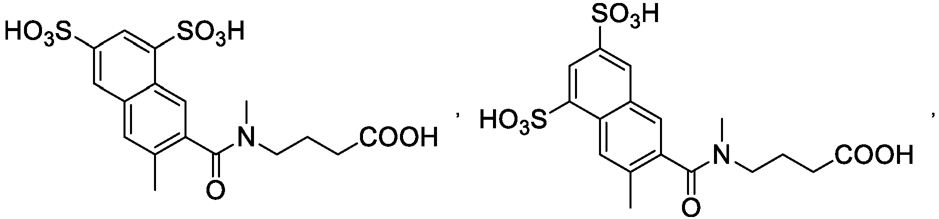

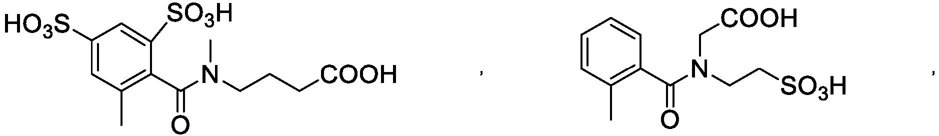

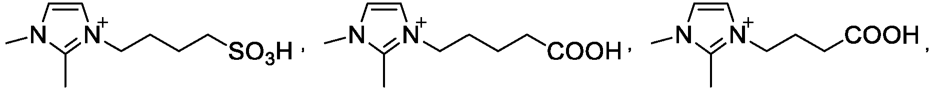

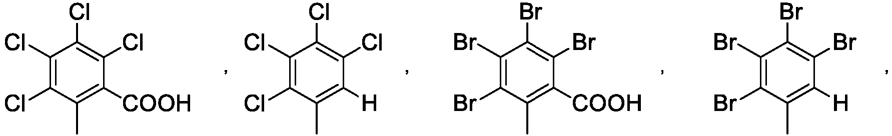

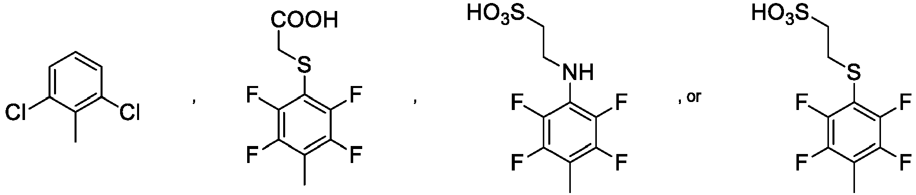

7. The dye of any one of claims 1-6, wherein R₂ is N(CH₃)₂.

8. The dye of any one of claims 1-7 comprising at least one SOsH group.

9. The dye of any one of claims 1-8 comprising at least one carboxyl group.

10. A compound having general formula (I), (II), (III) or (IV) 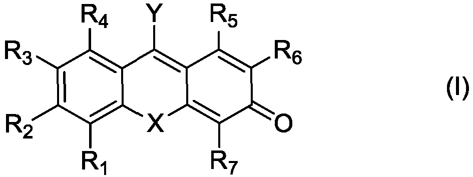 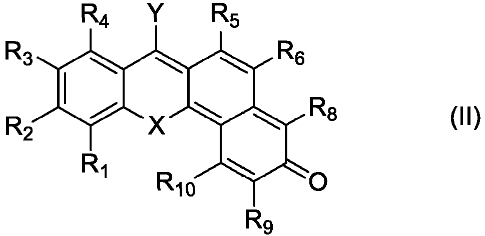 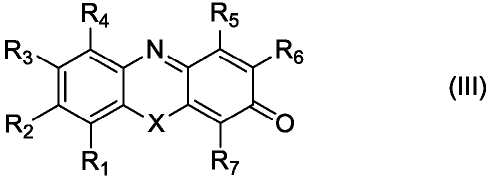 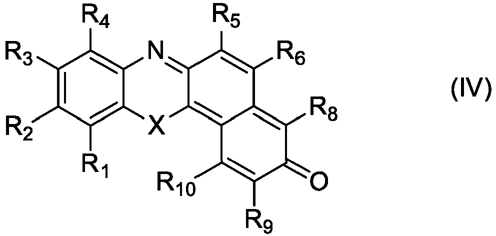 wherein
X = CR₁₁R₁₂, SiR₁₃R₁₄, sulfur, SO₂ or P(O)OR₁₅,
Y = hydrogen, a nitril group, a carboxyl group, a carboxylic acid derivative, an amino group or a straight-chain, branched or cyclic, saturated or unsaturated hydrocarbon moiety having up to 40 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, or CONR₃₁R₃₂;
R₁, R₃, R₄, R₅, R₆, R₇, R₈, R₉, R₁₀ independently are hydrogen, halogen, a hydroxyl group, thiol group, amino group, sulfo group, phospho group, nitro group, carbonyl group, carboxyl group, a carboxylic acid derivative, a nitrile group, isonitrile group, cyanate group, thiocyanate group, isothiocyanate group or a straight-chain, branched or cyclic saturated or unsaturated hydrocarbon moiety having up to 20 carbon atoms, which can contain or be substituted with one or more heteroatoms selected from the group of N, O, and S, or/and one or more substituents preferably selected from a group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂ and SOsH, or CONR₃₁R₃₂;
R₁₁, R₁₂, R₁₃, R₁₄, R₁₅, R₃₁, R₃₂ independently are hydrogen or a hydrocarbon group having 1-20 carbon atoms, wherein the hydrocarbon group optionally comprises one or more heteroatoms selected from the group of N, O, and S, or/and one or more substituents preferably selected from the group consisting of halogen, nitrile group, hydroxyl group, thiol group, amino group, nitro group, sulfo group, phospho group, carbonyl group, carboxyl group, a carboxylic acid derivative,
or R₁₁ and R₁₂ or R₁₃ and R₁₄, together with the atom to which they are attached, form a 3- to 7-membered ring, wherein the ring can comprise one or more double bonds or/and one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SOsH, CONR₃₁R₃₂ and a hydrocarbon group having from 1 - 20 carbon atoms or/and can be fused with one or more 3- to 7-membered rings, wherein the hydrocarbon group optionally comprises one or more heteroatoms selected from the group consisting of N, O and S or/and one or more substituents selected from the group consisting of halogen, OH, (O)alkyl, O(aryl), SH, S(alkyl), S(aryl), NH₂, NH(aryl), NH(alkyl), N(aryl)₂, N(alkyl)₂, NO₂, CHO, COOH, COO(alkyl), COO(aryl), PO₃H₂, SO₃H, or CONR₃₁R₃₂;
R₂ = NR₁₆R₁₇ or R₁ together with R₂ is 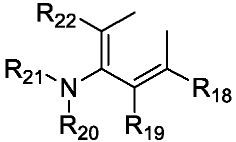
in which R₁₈, R₁₉ and R₂₂ are defined as R₁ and
R₁₆, R₁₇, R₂₀, R₂₁ are defined as R₁₁
or wherein at least one of R₁-R₂₂ forms a ring system with one or more neighboring moieties,
with the proviso,
that in a compound of formula (I) there are not simultaneous:
X = C(CH₃)₂;
R₂ = N(CH₃)₂; and
Y = 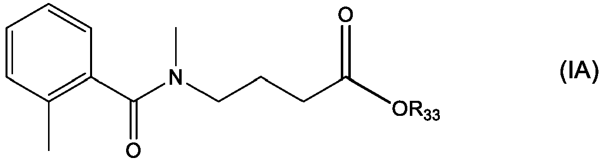
wherein R₃₃ = H or CH_{3;} and
that in a compound of formula (I) there are not simultaneous
X = C(CH₃)₂;
R₂ = N(CH₃)₂; and
Y = 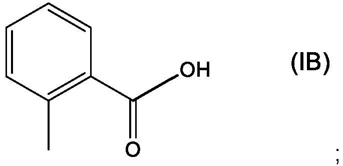 and
that in a compound of formula (I) there are not simultaneous
X = C(CH₃)₂;
R₂ = N(CH₃)₂; and
Y = 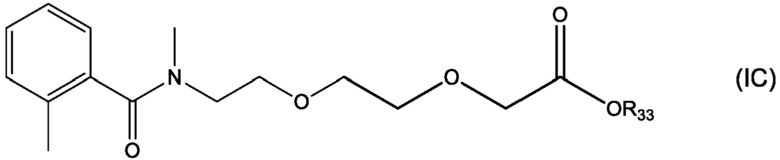
wherein R₃₃ = H or CH_{3;} and
that in a compound of formula (I) there are not simultaneous
X = Si(CH₃)₂;
R₂ = NH₂, NH(CH₃), N(CH₃)₂ or NH-C(O)CH₃; and
Y = 1-methyl-phenyl,
and wherein compounds of formula (III) with X = CR₁₁R₁₂ comprise at least one sulfo group.

11. Use of a dye of any one of claims 1-9 or of a compound of claim 10 as pH sensor.

12. Wound dressing comprising a dye of any one of claims 1-9 and/or a compound of claim 10.

13. Use of a dye of any one of claims 1-9 or of a compound of claim 10 as marker group for the qualitative and/or quantitative determination of an analyte in a sample.

14. Use of a dye of any one of claims 1-9 or of a compound of claim 10 as a pH sensor according to claim 11 or as a marker group according to claim 13 comprising bonding the dye or compound to the analyte or a substrate.

15. A method of preparing a dye or compound having formula (I), (II), (III) or (IV) as defined in any one of claims 1-10 comprising the steps:
a) conversion of a precursor compound by a one-step one-pot synthesis using an aqueous alkaline or alkaline earth hydroxide solution as reaction medium.
b) using lithium, sodium, potassium, cesium, calcium, or barium hydroxide or mixtures of different hydroxides.
c) using a temperature of at least 0°C, more preferably a temperature range of 25 - 100°C or even more preferably of 50 - 85°C and
d) employing a co-solvent, preferably selected from the group of acetone, acetonitrile, ethanol, or methanol.
